# EUROPEAN PATENT APPLICATION

(11) **EP 2 940 149 A1**
(43) Date of publication of application: **04.11.2015**
(21) Application number: 13869328.8
(22) Date of filing: 24.12.2013
(51) Int. Cl.: C12Q 1/68, C12N 15/09

(54) **DETECTION METHOD FOR HYDROXYMETHYLATED CYTOSINE IN DNA AND REAGENT KIT FOR DETECTION**

(30) Priority: 28.12.2012 JP 2012286925
(71) Applicant: Wako Pure Chemical Industries, Ltd., Osaka-shi Osaka 540-8605 (JP)
(72) Inventor: HAYASHIDA, Yukinobu, Amagasaki-shi Hyogo 661-0963 (JP); YAMAMOTO, Naoyuki, Amagasaki-shi Hyogo 661-0963 (JP)
(74) Representative: Winter, Brandl, Fürniss, Hübner, Röss, Kaiser, Polte - Partnerschaft mbB
(86) International application number: PCT/JP2013/084413
(87) International publication number: WO 2014/103980

(57) **Abstract**

The present invention is to provide a method for detecting a hydroxymethylated cytosine in DNA and a detection kit therefor. The present invention relates to "a method for detecting the hydroxymethylated cytosine in DNA, which the method comprises: (1) a step in which a single-stranded DNA is contacted with (i) a polyvalent metal oxide or a polyvalent metal acid salt of a metal atom selected from group 6, group 8, group 9 and group 10 of the periodic table, and contacted with (ii) a peroxide selected from persulfuric acid, percarboxylic acids and the salts thereof; (2) a step in which a specific region of the single-stranded DNA treated in (1) is subjected to amplification treatment; (3) a step in which the presence or absence of an objective amplification product obtained in (2) is detected; and (4) a step in which on the basis of the results of (3), the presence or absence of hydroxymethylated cytosine in the specific region of DNA is determined.", "a reagent kit for detecting hydroxymethylated cytosine in DNA comprisingof a reagent including the above polyvalent metal oxide or a polyvalent metal acid salt and the above peroxide".

## Description

### TECHNICAL FIELD

The present invention relates to a method for detecting a hydroxymethylated cytosine in DNA and a reagent kit for the detection.

### BACKGROUND ART

Cytosine which is one of the bases that constitute a DNA assuming the genetic information of the living organism is methylated by DNA methyl transferase (DMNT), and by the methylation in a promoter region that is a typical gene expression control mechanism of epigenetics, gene expression is supressed. On the other hand, the methylated cytosine (hereinafter, it may be abbreviated as "mC") is hydroxylated by a hydroxylase such as Ten-eleven translocation (TET) family to a hydroxymethylated cytosine (hereinafter, it may be abbreviated as "hmC"), which is considered to promote the gene expression, therefore, the identification of cytosine, mC and hmC in DNA is considered to provide a clue to investigate the expression status of genetic information.

On the other hand, as the detection method of mC in DNA, a bisulfite method (Patent Literature 1) has been known, but it is not possible to distinguish hmC from mC by this method. In addition, as the detection method of hmC that can distinguish between the two, the enzymatic treatment method (Non-Patent Literature 1), the immunoprecipitation method (Non-Patent Literature 2), the oxidation method (Non-Patent Literature 3, Patent Literature 2) and the like have been known.

The enzymatic treatment method is a method in which the DNA to be detected is glucosylated by using T4-BGT which is an enzyme that specifically transfers glucose from UDP glucose to 5-hmC in a 5-hmC residue, then by using a restriction enzyme Mspl which recognizes a sequence of CCGG, the restriction enzyme treatment is carried out, and by subjecting said DNA to an amplification treatment by PCR, presence or absence of the hmC in the DNA to be detected is determined from the presence or absence of the obtained amplification product.

The immunoprecipitation method is a method for detecting the presence or absence of hmC in DNA to be detected in which the genomic DNA is fragmented by ultrasonic disruption or restriction enzyme treatment, followed by immunoprecipitation reaction using anti-hmC antibody, then by carrying out sequence analysis of the DNA fragment bound with the anti-hmC antibody by microarray method, the presence or absence of hmC in the DNA to be detected is detected.

The oxidation method is a method in which a synthesized single-stranded DNA is oxidized by bringing into contact with sodium tungstate and hydrogen peroxide simultaneously, and then the sequence analysis of the DNA is carried out using a sequencer. By the above oxidation reaction, the hydrolysis reaction of the amino group at the 4-position of cytosine is taken place, and thereby, the hmC is changed into a thymine derivative. Therefore, when the analysis of DNA sequence is carried out using the single-stranded DNA after the oxidation reaction as a template, adenine is introduced in the complementary strand side where the hmC has been present before oxidation reaction. On the other hand, since such oxidation reaction does not proceed in cytosine and mC, even after the oxidation reaction, guanine is introduced into the cytosine and mC, as well as the complementary strand side of the hmC which is not subjected to oxidation reaction. That is, by performing such oxidation reaction, and by comparing the results of sequence analysis of DNA before and after the oxidation reaction, the presence or absence of hmC in DNA can be detected, and its location can be specified.

### CITATION LIST

### PATENT LITERATURE

Patent Literature 1: WO2013/089063 A1
Patent Literature 2: WO2012/141324 A1

### NON-PATENT LITERATURE

Non-Patent Literature 1: Nature 466, 1129-1133 (2010)
Non-Patent Literature 2: Nature Biotechnolo. 29, 68-72 (2011)
Non-Patent Literature 3: Chem. Comm. 47, 11231-11233 (2011)

### SUMMARY OF INVENTION

### TECHNICAL PROBLEM

In the conventional method described above for detecting hmC in DNA, there are problems as described below. The present invention is intended to provide a method for detecting hmC in DNA and a kit therefor which resolves these problems.

That is, in the enzymatic treatment method, there are problems that (i) because of using restriction enzyme MspI, it is necessary to set the optimum conditions for the enzyme, which requires a complicated operation; (ii) the restriction enzyme MspI recognizes CCGG sequence in DNA, however, frequency of appearance of this recognition sequence in the DNA to be detected is low, in addition, in the DNA not having the recognition sequence, the presence or absence of hmC cannot be detected; (iii) since there are some cases where cleavage of DNA by the enzyme is insufficient, reproducibility is low; and so on.

In addition, the immunoprecipitation method is a method of detecting hmC in distinction from mC using anti hmC antibody, however, there is a problem that because of low specificity for hmC of said antibody, distinction in high accuracy cannot be achieved.

In the oxidation method, there are problems that (i) in a single-stranded synthetic DNA of about 50 bases including only one base of hmC, the rate of conversion from hmC to thymine derivative is low as about 60% to 80%, therefore the detection of hmC is not accurate; (ii) on the occasion of DNA sequencing, it is necessary to clone the sequence of interest, a complicated operation has to be carried out; and the like.

### SOLUTION TO PROBLEM

The present inventors have studied intensively to solve the problems by the above conventional methods, and as a result, the present inventors have found that by bringing the single-stranded DNA into contact with a polyvalent metal oxide or a polyvalent metal acid salt of a metal selected from group 6, group 8, group 9 and group 10 of the periodic table (hereinafter, these it may be abbreviated as "polyvalent metal oxides pertaining to the present invention"), anda peroxide selected from persulfuric acid, percarboxylic acids and the salts thereof (hereinafter, these may be abbreviated as "peroxide pertaining to the present invention") and performing oxidation reaction, only hmC can be altered to an oxide which does not go into the DNA amplification reaction, in other words, in cytosine and mC which have been subjected to said oxidation reaction, the amplification reaction proceeds, and in an oxide of hmC which has been subjected to said oxidation reaction, the amplification reaction does not proceed, and have thus completed the present invention.

That is, the constitution of the present invention consists of the following constituents.
"1. A method for detecting a hydroxymethylated cytosine in DNA, whichcomprises following steps (1) to (4):
   (1): a step in which a single-stranded DNA is contacted with (i) a polyvalent metal oxide or a polyvalent metal acid salt of a metal atom selected from group 6, group 8, group 9 and group 10 of the periodic table and (ii) contacted with a peroxide selected from persulfuric acid, percarboxylic acids and salts thereof;
   (2): a step in which a specific region of the single-stranded DNA treated in (1) is subjected to amplification treatment;
   (3): a step in which the presence or absence of an objective amplification product obtained in (2) is detected; and
   (4): a step in which on the basis of the results of (3), the presence or absence of hydroxymethylated cytosine in the Specific region of DNA is determined."
"2. A reagent kit for detecting hydroxymethylated cytosine in DNA, comprisinga reagent including a polyvalent metal oxide pertaining to the present invention and a reagent including peroxide pertaining to the present invention."

### ADVANTAGEOUS EFFECTS OF INVENTION

According to the method of the present invention, it is possible to detect hmC in DNA simply and accurately. That is, the method of the present invention does not have such problems as described above in the enzymatic treatment method, immunoprecipitation method and oxidation method which are the conventional methods, and does not require DNA sequencing, therefore, the hmC in DNA can be detected simply.

### BRIEF DESCRIPTION OF DRAWINGS

Fig. 1 is a figure showing classification of DNA oxidation step pertaining to the present invention.
Fig. 2 is a figure showing the relationship between objective amplification product, specific region of single-stranded DNA, corresponding region of the complementary strand, detection target region, primer sequence and adapter sequence pertaining to the present invention.
Fig. 3 is a figure showing the results of agarose gel electrophoresis of the products obtained by subjecting various DNA to the PCR in Example 1 to 2.
As a polyvalent metal oxide pertaining to the present invention, (A) represents the results obtained when sodium tungstate is used, and (B) represents the results obtained when potassium tungstate is used, respectively.

Lane 1 represents the result when a marker is used; lane 2, 5, 8 and 11 represent the results when DNA including cytosine is used; lane 3, 6, 9 and 12 represent the results when DNA including mC is used, respectively.lane 4, 7, 10 and 13 represent the results when DNA including hmC is used, respectively. It should be noted that, lane 2 to lane 4 represent the electrophoretic pattern of the products carried out PCR of 15 cycles; lane 5 to lane 7 represent the electrophoretic pattern of the products carried out PCR of 20 cycles, lane 8 to lane 10 represent the electrophoretic pattern of the products carried out PCR of 30 cycles; and lane 11 to lane 13 represent the electrophoretic pattern of the products carried out PCR of 35 cycles.

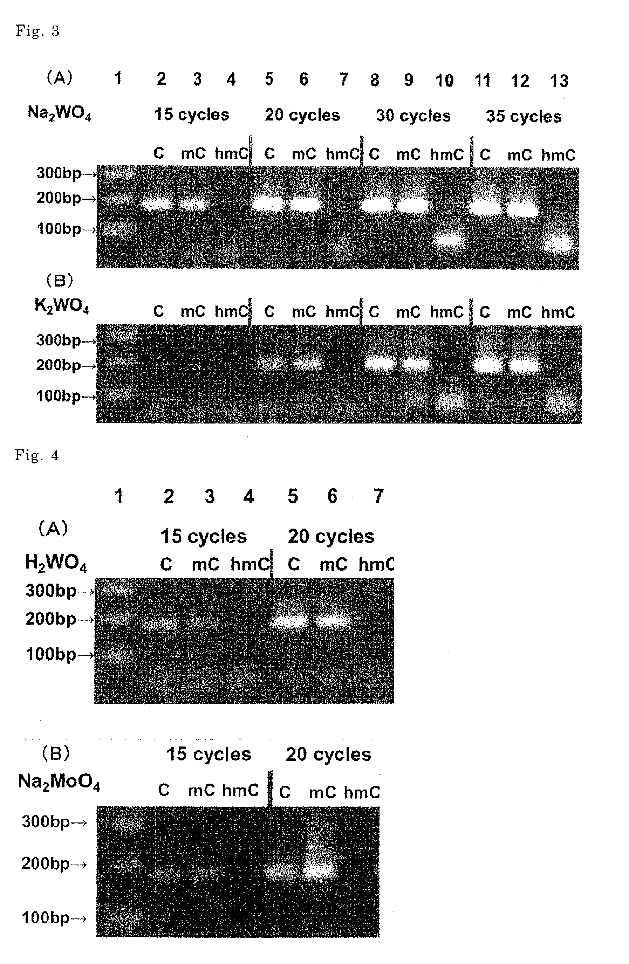

Fig. 4 is a figure showing the agarose gel electrophoresis of the products obtained by subjecting various DNA to the PCR in Example 3 to 4.
As a polyvalent metal oxides of the present invention, (A) represents the results obtained when tungstic acid is used, and (B) represents the results obtained when sodium molybdate is used, respectively.

Lane 1 represent the electrophoretic pattern of marker, lane 2 to lane 4 represent the electrophoretic pattern of the products carried out PCR of 15 cycles, lane 5 to lane 7 represent the electrophoretic pattern of the products carried out PCR of 20 cycles.

Lane 2 and 5 represent the results when DNA including cytosine is used; lane 3 and 6 represent the results when DNA including mC is used; lane 4 and 7 represent the results when DNA including hmC is used, respectively.

### DESCRIPTION OF EMBODIMENTS

### 1. Polyvalent metal oxides pertaining to the present invention

In polyvalent metal oxides pertaining to the present invention, the valence of the metal is usually 2 or more by absolute value, preferably 2 to 8, more preferably 6.

Such metal atom includes molybdenum such as molybdenum (II), molybdenum (III), molybdenum (IV), molybdenum (V), molybdenum (VI), and molybdenum (-II); tungsten such as tungsten (VI), tungsten (V) tungsten (IV), tungsten (III), tungsten (II), and tungsten (-II); ruthenium such as ruthenium (VIII), ruthenium (VII), ruthenium (VI), ruthenium (IV), ruthenium (III), ruthenium (II), and ruthenium (-II); osmium such as osmium (VIII), osmium (VII), osmium (VI), osmium (V), osmium (IV), osmium (III), and osmium (II); rhodium such as rhodium (VI), rhodium (V), rhodium (IV), rhodium (III), and rhodium (II), iridium such as iridium (VI), iridium (V), iridium (IV), iridium (III), iridium (II), and iridium (-III); palladium such as palladium (VI), palladium (IV), palladium (II); platinum such as platinum (VI), platinum (V), platinum (IV), platinum (III), platinum (II), and platinum (-II); nickel such as nickel (II), and nickel (III), cobalt such as cobalt (II) and cobalt (III); chromium such as chromium (VI), chromium (V), chromium (IV), chromium (III), chromium (II), and chromium (-II) and the like; and molybdenum such as molybdenum (II), molybdenum (III), molybdenum (IV), molybdenum (V), molybdenum (VI), and molybdenum (-II); tungsten such as tungsten (VI), tungsten (V), tungsten (IV), tungsten (III), tungsten (II), and tungsten (-II) are preferable, and molybdenum (VI) and tungsten (VI) are more preferable, and tungsten (VI) is particularly preferable.

In addition, the polyvalent metal oxide pertaining to the present invention includes, for example, chromium oxide (II) (CrO), chromium oxide (III) (Cr₂O₃), chromium oxide (IV) (CrO₂), chromium oxide (VI) (CrO₃), molybdenum oxide (IV) (MoO₂), molybdenum oxide (VI) (MoO₃), tungsten oxide (III) (W₂O₃), tungsten oxide (IV) (WO₂), tungsten oxide (VI) (WO₃), ruthenium oxide (IV) (RuO₂), ruthenium oxide (VIII) (RuO₄), osmium oxide (IV) (OsO₂), osmium oxide (VIII) (OsO₄), rhodium oxide (III) (Rh₂O₃), rhodium oxide (IV) (RhO₂), iridium oxide (III) (Ir₂O₃), iridium oxide (IV) (IrO₂), palladium oxide (II) (PdO), nickel oxide (II), nickel oxide (III), cobalt oxide (II), cobalt oxide (III) and platinum oxide (II) (PtO), tungstic acid (H₂WO₄), and the like.

The polyvalent metal acid salts pertaining to the present invention means, for example, the salts of polyvalent metal acid with an alkali metal or the salts with alkaline earth metal. Said polyvalent metal acid includes, for example, tungstic acid (H₂WO₄), molybdenum acid (H₂MoO₄) and the like, and, tungstic acid (H₂WO₄) is preferable. Said alkali metal includes, lithium, sodium, potassium, rubidium and the like, and said alkaline earth metal includes, calcium, strontium, barium, and radium and the like.

The salt with an alkali metal includes, for example, sodium tungstate (Na₂WO₄), potassium tungstate (K₂WO₄), and sodium molybdate (Na₂MoO₄) and the like, and, sodium tungstate (Na₂WO₄), potassium tungstate (K₂WO₄) and the like are preferable.

The salt with an alkaline earth metal includes, for example, calcium tungstate (CaWO₄), magnesium tungstate (MgWO₄), strontium tungstate (SrWO₄), barium tungstate (BaWO₄), calcium molybdate (CaMoO₄), magnesium molybdate (MgMoO₄), strontium molybdate (SrMoO₄) and the like.

Preferred example of the polyvalent metal oxide pertaining to the present invention include, among the above-described specific examples, sodium tungstate (Na₂WO₄), potassium tungstate (K₂WO₄), sodium molybdate (Na₂MoO₄) and tungstic acid (H₂WO₄) and the like, and, sodium tungstate (Na₂WO₄), potassium tungstate (K₂WO₄) and tungstic acid (H₂WO₄) are more preferable.

### 2. Peroxide pertaining to the present invention

The peroxide pertaining to the present invention is the one which is selected from persulfuric acid, percarboxylic acids and the salts thereof.

The above percarboxylic acid is an organic compound having a carboxy group, and a peracid in which a hydroxy group (-OH) of the carboxy group is replaced by hydroperoxy group (-OOH), and includes, for example, benzoyl peroxide, peracetic acid and the like.

The salt of the above persulfuric acid and the salt of the above percarboxylic acid include, for example, the salt with alkali metals, and specifically, include sodium hydrogen persulfate, sodium persulfate, potassium hydrogen persulfate, potassium persulfate, sodium peracetate, potassium peracetate, and the like.

Among peroxide pertaining to the present invention, the preferred one includes sodium hydrogen persulfate, potassium hydrogen persulfate, and the like.

### 3. DNA pertaining to the present invention

DNA capable of detecting the presence or absence of hmC in the present invention may be the DNA synthesized chemically, or the DNA extracted from living organisms. The DNA extracted from living organisms includes DNA extracted by a method known per se such as the alkali SDS method described, for example, in "Labo Manual for Genetic Engineering" (Maruzen Co., Ltd.) and "Handbook for Genetic Engineering" (Yodosha Co., Ltd.)), etc. In addition, the DNA extracted from cells, microorganisms, viruses and the like by using a commercially available extraction kit for genomic DNA can also be used as a DNA extracted from living organisms.

The number of base pair of the DNA pertaining to the present invention is usually 60 to 500, preferably 60 to 300, and the number of nucleotides of single-stranded DNA pertaining to the present invention is usually 60 to 500, preferably 60 to 300.

### 4. Method of the present invention

The present invention is a method for detecting hmC in DNA, and is characterized in that the method comprises the following steps (1) to (4).
(1): A step in which a single-stranded DNA is contacted with a polyvalent metal oxide pertaining to the present invention and with a peroxide pertaining to the present invention (DNA oxidation step);
(2): a step in which a specific region of the single-stranded DNA treated in (1) is subjected to amplification treatment (DNA amplification step);
(3): a step in which the presence or absence of an objective amplification product obtained in (2) is detected (amplification product detection step), and
(4): a step in which, on the basis of the results of (3), the presence or absence of hydroxymethylated cytosine in the specific region of DNA is determined (determination step).

### (1) DNA oxidation step [step (1)]

The DNA oxidation step [step (1)] is a step (treatment) in which the hmC in a single-stranded DNA is oxidized by bringing the single-stranded DNA into contact with the polyvalent metal oxides pertaining to the present invention and the peroxide pertaining to the present invention.

In the DNA oxidation step pertaining to the invention, finally, oxidation reaction may be taken place by contacting the polyvalent metal oxides pertaining to the present invention and the peroxide pertaining to the present invention with single-stranded DNA, and for example, the following method (I) and method (II) are included, and the method (I) is preferable.
(I): The method in which after the single-stranded DNA is contacted with polyvalent metal oxides pertaining to the present invention, further contacted with the peroxide pertaining to the present invention.
(II): The method in which single-stranded DNA is contacted with the polyvalent metal oxides pertaining to the present invention and the peroxide pertaining to the present invention, simultaneously.

Use concentration of the polyvalent metal oxides pertaining to the present invention is, as a final concentration in the reaction solution in contacting with the single-stranded DNA, usually, the lower limit is 100 mM or more, preferably 200 mM or more, and the upper limit is 3000 mM or less, preferably 2000 mM or less.

Use concentration of the peroxide pertaining to the present invention is, as a final concentration in the reaction solution in contacting with the single-stranded DNA, usually, the lower limit is 10 mM or more, preferably 50 mM or more, and the upper limit is 300 mM or less, preferably 200 mM or less.

As the amount of single-stranded DNA in the reaction solution in contacting the single-stranded DNA pertaining to the present invention with the polyvalent metal oxides pertaining to the present invention and/or the peroxide pertaining to the present invention is, in 100 µL reaction solution, usually, the lower limit is 50 ng or more, preferably 60 ng or more, and the upper limit is 200 ng or less, preferably 190 ng or less.

In order to bring the single-stranded DNA into contact with the polyvalent metal oxides pertaining to the present invention, incubation may be carried out, for example, under the lower limit usually at 15°C or higher, preferably at 20°C or higher, and the upper limit usually at 90°C or lower, preferably at 45°C or lower, for usually 5 minutes to 480 minutes, preferably for 10 minutes to 60 minutes. It should be noted that, the above polyvalent metal oxides pertaining to the present invention may be used appropriately as a mixture of two or more kinds thereof.

When the single-stranded DNA is contacted with the peroxide pertaining to the present invention, incubation may be carried out, for example, under the lower limit usually at 30°C or higher, preferably at 50°C or higher, and the upper limit usually at 100°C or lower, preferably at 90°C or lower, most preferably at 70°C or lower, and usually for 30 minutes to 480 minutes, preferably for 120 minutes to 300 minutes. It should be noted that, the above peroxide pertaining to the present invention may be used appropriately as a mixture of two or more kinds thereof.

When the single-stranded DNA is contacted with the polyvalent metal oxides pertaining to the present invention and the peroxide pertaining to the present invention simultaneously, incubation may be carried out, for example, at the lower limit usually 30°C or higher, preferably at 50°C or higher, and the upper limit usually at 100°C or lower, preferably at 90°C or lower, most preferably at 70°C or lower, and usually for 30 minutes to 480 minutes, preferably for 120 minutes to 300 minutes. It should be noted that, the polyvalent metal oxides pertaining to the present invention and the peroxide pertaining to the present invention may be used appropriately as a mixture of two or more kinds thereof.

In the case where the DNA pertaining to the present invention is consisted of two or more strands, single-stranded DNA obtained by performing a single strand formation treatment well-known per se may be used.

The single strand formation treatment of said DNA may be any method as long as it is usually used in this field, there are included, for example, heat treatment, alkali treatment, and the treatment with chaotropic agents such as urea, and the like.

Among the above treatment, the heat treatment is performed by carrying out incubation of a solution (hereinafter, referred to as "DNA solution") of the DNA dissolved in a solvent such as water, Good's buffer solution such as MES and HEPES, phosphate buffer solution, Tris buffer solution, glycine buffer solution, borate buffer solution, sodium hydrogen carbonate buffer solution, under the lower limit usually at 85°C or higher, preferably at 90°C or higher, and the upper limit usually at 100°C or lower, preferably at 95°C or lower, and usually for 30 seconds to 30 minutes, preferably for 1 minute to 5 minutes. It should be noted that, said DNA solution may include the polyvalent metal oxides pertaining to the present invention and the peroxide pertaining to the present invention.

The above alkali treatment is carried out, for example, by adding alkali or its aqueous solution to the DNA solution, and by making said DNA solution alkaline of usually pH 10 to pH 14, preferably pH 12 to pH 14. The alkali used in such purpose includes, for example, alkali metal hydroxide such as sodium hydroxide and potassium hydroxide; alkaline-earth metal hydroxide such as barium hydroxide, magnesium hydroxide, and calcium hydroxide; alkaline metal carbonate such as sodium carbonate; ammonia, and amines and the like, however, among them, alkali metal hydroxide such as sodium hydroxide and potassium hydroxide is preferable, and sodium hydroxide is particularly preferable among these. Said alkaline treatment is performed, more specifically, by adding usually 0.1 µL to 1 µL, preferably 0.1 µL to 0.5 µL of 0.5 M to 3 M aqueous alkaline solution to 1 µL of DNA solution including usually 1 ng to 300 ng, preferably 50 ng to 200 ng of DNA, and by reacting usually for 5 minutes to 60 minutes, preferably for 5 minutes to 30 minutes at usually 25°C to 70°C, preferably at 30°C to 50°C.

In the single strand formation treatment, among the above treatments, the alkaline treatment is preferable because the possibility of single-stranded DNA going back to double-stranded is low on the occasion of shifting to the next step. In addition, in the case of using naturally occurring genomic DNA, the alkali treatment is preferable as compared to the heat treatment, from the point that the damage to genomic DNA by alkali treatment is small.

The above treatment with chaotropic agents such as urea may be carried out according to the method well-known per se.

The single-strand formation treatment pertaining to the present invention may be carried out simultaneously with the aforementioned DNA oxidation step. That is, the single-strand formation treatment may be carried out simultaneously with the step of bringing the single-stranded DNA into contact with the polyvalent metal oxides pertaining to the present invention, and the single-strand formation treatment may be carried out simultaneously with the step of bringing the single-stranded DNA into contact with the polyvalent metal oxides pertaining to the present invention and the peroxide pertaining to the present invention.

The specific method for DNA oxidation step pertaining to the present invention is described by classifying into the following three cases:
(A) The case where a single-stranded DNA is contacted with the polyvalent metal oxides pertaining to the present invention and the peroxide pertaining to the present invention (the case where a complementary strand of said single-stranded DNA is not present);
(B) The case where after the DNA consisting of two or more strands is subjected to the single strand formation treatment, said single-stranded DNA is contacted with the polyvalent metal oxides pertaining to the present invention and the peroxide pertaining to the present invention (the case where a complementary strand of said single-stranded DNA is present);
(C) The case where at the same time of the single strand formation treatment of the DNA consisting of two or more strands, the DNA is contacted with the polyvalent metal oxides pertaining to the present invention and the peroxide pertaining to the present invention (the case where a complementary strand of said single-stranded DNA is present).

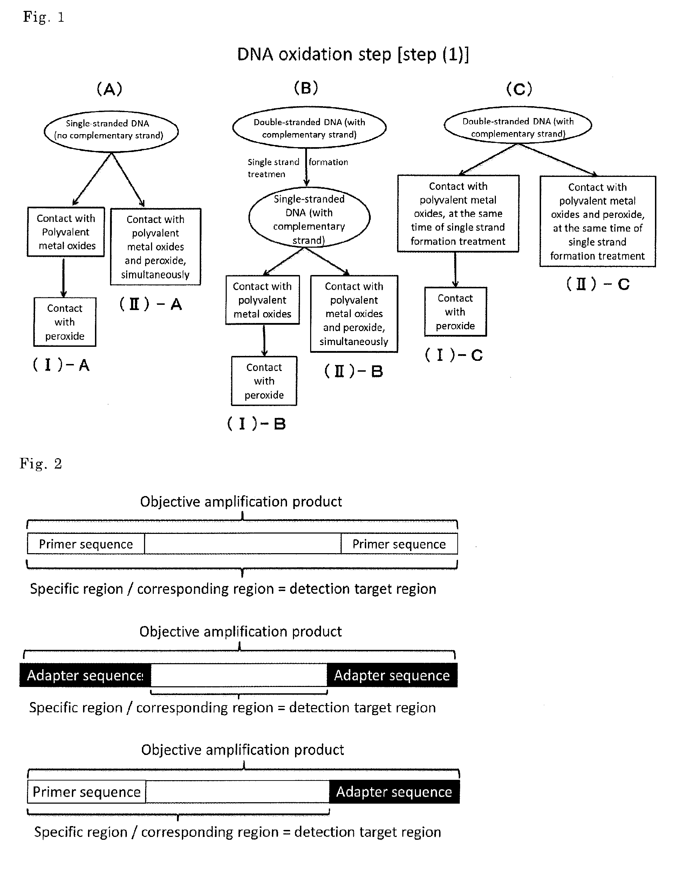

It should be noted that outline of each case of the above (A) to (C) is summarized in Fig. 1.

The above case (A) is explained in detail below. As shown in Fig. 1, the above (A) is divided into the case (I)-A and the case (II)-A below and each of these may be carried out as follows:

### (I)-A. The case where the single-stranded DNA is contacted with the peroxide pertaining to the present invention after contacting with the polyvalent metal oxides pertaining to the present invention

For example, to a solution including 50 ng to 200 ng of the above single-stranded DNA, an aqueous solution including polyvalent metal oxides pertaining to the present invention is added and mixed so that the final concentration after mixing will be 100 mM to 3000 mM, preferably 200 mM to 2000 mM, and incubation is carried out under the lower limit usually at 15°C or higher, preferably at 20°C or higher, and the upper limit usually at 60°C or lower, preferably at 45°C or lower, and usually for 5 minutes to 480 minutes, preferably for 10 minutes to 60 minutes (referred to as solution 1).

After that, to the solution 1, an aqueous solution including peroxide pertaining to the present invention is added so that the final concentration after mixing will be 10 mM to 300 mM, preferably 50 mM to 200 mM and mixed, and incubation is carried out usually at 30°C to 100°C, preferably at 50°C to 70°C, and usually for 30 minutes to 480 minutes, preferably for 120 minutes to 300 minutes.

It should be noted that the liquid volume of the above overall reaction is made to be usually 50 µL to 100 µL.

### (II)-A. The case where the single-stranded DNA is contacted with the peroxide pertaining to the present invention and the polyvalent metal oxides pertaining to the present invention simultaneously

For example, to a solution including 50 ng to 200 ng of the above single-stranded DNA, an aqueous solution including polyvalent metal oxides pertaining to the present invention and an aqueous solution containing peroxide pertaining to the present invention are added and mixed so that the final concentration of the polyvalent metal oxides pertaining to the present invention after mixing will be 100 mM to 3000 mM, preferably 200 mM to 2000 mM, and also, the final concentration of the peroxide pertaining to the present invention after mixing will be 10 mM to 300 mM, preferably 50 mM to 200 mM, and incubation is carried out under the lower limit usually at 30°C or higher, preferably at 50°C or higher, and the upper limit usually at 90°C or lower, preferably at 70°C or lower, and for 30 minutes to 480 minutes, preferably for 120 minutes to 300 minutes.

It should be noted that the liquid volume of the above overall reaction is made to be usually 50 µL to 100 µL.

The above case (B) is explained in detail below. The above (B) is divided into the following (I)-B1, (I)-B2, (II)-B1, and (II)-B2, and each of these may be carried out as follows:
It should be noted that, (I)-B1 and (II)-B1 are the case where the single strand formation is performed by alkali treatment, and (I)-B2 and (II)-B2 are the case where the single strand formation is performed by heat treatment, and these treatments are performed according to the method of the treatment for single strand formation of DNA well-known per se.

### (I)-B1. The case where after the DNA consisting of 2 or more strands is subjected to the alkali treatment, said single-stranded DNA is contacted with the polyvalent metal oxides pertaining to the present invention and the peroxide pertaining to the present invention

For example, to a solution including 50 ng to 200 ng of the above DNA consisting of 2 or more strands, 20 mol to 200 mol, preferably 50 mol to 100 mol alkali solution is added, thereby pH of the solution including DNA is made pH 10 to pH 14, preferably pH 12 to pH 14, and incubation is carried out usually for 5 minutes to 60 minutes, preferably for 5 minutes to 30 minutes, usually at 25°C to 70°C, preferably at 30°C to 50°C, thus the treatment for single strand formation of DNA by alkali treatment is carried out (referred to as (I)-B1 solution 1).

After that, using the above "(I)-B1 solution 1" instead of the above "a solution including 50 ng to 200 ng of single-stranded DNA", and according to the method described in (I)-A, single-stranded DNA in the "(I)-B1 solution 1" is contacted firstly with the polyvalent metal oxides pertaining to the present invention and then with the peroxide pertaining to the present invention.

### (I)-B2. The case where after the DNA consisting of 2 or more strands is subjected to heat treatment, said single-stranded DNA is contacted with the polyvalent metal oxides pertaining to the present invention and the peroxide pertaining to the present invention

For example, a solution containing 50 ng to 200 ng of the above DNA consisting of 2 or more strands is subjected to single strand formation treatment by heat treatment of DNA, usually at 85°C or higher, preferably at 90°C or higher, and the upper limit usually at 100°C or lower, preferably at 95°C or lower, and usually for 30 seconds to 30 minutes, preferably for 1 minute to 5 minutes (referred to as (I)-B2 solution 1).

After that, using the above "(I)-B2 solution 1" instead of the above "a solution including 50 ng to 200 ng of single-stranded DNA", and according to the method described in (I)-A, single-stranded DNA in the "(I)-B2 solution 1" is contacted firstly with the polyvalent metal oxides pertaining to the present invention and then with the peroxide pertaining to the present invention.

### (II)-B1. The case where the DNA consisting of 2 or more strands is subjected to alkali treatment, then contacted with polyvalent metal oxides pertaining to the present invention and peroxide pertaining to the present invention simultaneously

For example, to a solution including 50 ng to 200 ng of the above DNA consisting of 2 or more strands, 20 mol to 200 mol, preferably 50 mol to 100 mol alkali solution is added to make the pH of the solution including DNA pH 10 to pH 14, preferably pH 12 to pH 14, and incubation is carried out usually for 5 minutes to 60 minutes, preferably for 5 minutes to 30 minutes, usually at 25°C to 70°C, preferably at 30°C to 50°C, thus the treatment for single strand formation of DNA by alkali treatment is carried out (referred to as (II)-B1 solution 1).

After that, using the above "(II)-B1 solution 1" instead of the above "a solution including 50 ng to 200 ng of single-stranded DNA", and according to the method described in (II)-A, the single-stranded DNA in the "(II)-B1 solution 1" is contacted with the polyvalent metal oxides pertaining to the present invention and the peroxide pertaining to the present invention simultaneously.

### (II)-B2. The case where the DNA consisting of 2 or more strands is subjected to heat treatment, then contacted with the polyvalent metal oxides pertaining to the present invention and the peroxide pertaining to the present invention simultaneously

For example, a solution including 50 ng to 200 ng of the aforementioned DNA consisting of 2 or more strands is subjected to single strand formation treatment by heat treatment of DNA, usually at 85°C or higher, preferably at 90°C or higher, and the upper limit usually at 100°C or lower, preferably at 95°C or lower, and usually for 30 seconds to 30 minutes, preferably for 1 minute to 5 minutes, thus the DNA single strand formation by heat treatment is carried out (to be used as (II)-B2 solution 1).

After that, using the above "(II)-B2 solution 1" instead of the above "a solution including 50 ng to 200 ng of single-stranded DNA", and according to the method described in (II)-A, the single-stranded DNA in the "(II)-B2 solution 1" is contacted with the polyvalent metal oxides pertaining to the present invention and the peroxide pertaining to the present invention simultaneously.

The above case (C) is explained in detail below. The above (C) is divided into the following (I)-C1, (I)-C2, (II)- C1, and (II)- C2 and each of these may be carried out as follows:
It should be noted that, (I)-C1 and (II)-C1 are the case where the single strand formation is performed by alkali treatment, and (I)-C2 and (II)-C2 are the case where the single strand formation is performed by heat treatment, and these treatments are performed according to the method of treatment for single strand formation of DNA well-known per se.

### (I)-C1. The case where at the same time as the alkali treatment of DNA consisting of 2 or more strands, the DNA is contacted with the polyvalent metal oxides pertaining to the present invention, and then contacted with the peroxide pertaining to the present invention

For example, to a solution containing 50 ng to 200 ng of the above DNA consisting of 2 or more strands, an aqueous solution including polyvalent metal oxides pertaining to the present invention is added and mixed so that the final concentration of the polyvalent metal oxides pertaining to the present invention after mixing will be 100 mM to 3000 mM, preferably 200 mM to 2000 mM, and to this solution by adding 20 mol to 200 mol, preferably 50 mol to 100 mol alkali solution to make the pH of the solution containing DNA pH 10 to pH 14, preferably pH 12 to pH 14, then incubation is carried out under the lower limit usually at 15°C or higher, preferably at 30°C or higher, and the upper limit usually at 60°C or lower, preferably at 45°C or lower, and usually for 5 minutes to 480 minutes, preferably for 10 minutes to 60 minutes (referred to as (I)-C1 solution 1).

After that, using the above "(I)-C1 solution 1" instead of the above solution 1, and according to the method described in (I)-A, the single-stranded DNA in the "(I)-C1 solution 1" is contacted with the peroxide pertaining to the present invention.

### (I)-C2. The case where at the same time as the heat treatment of DNA consisting of 2 or more strands, the DNA is contacted with the polyvalent metal oxides pertaining to the present invention, and then contacted with the peroxide pertaining to the present invention

For example, to a solution including 50 ng to 200 ng of the aforementioned DNA consisting of 2 or more strands, an aqueous solution including polyvalent metal oxides pertaining to the present invention is added and mixed so that the final concentration of the polyvalent metal oxides pertaining to the present invention after mixing will be 100 mM to 3000 mM, preferably 200 mM to 2000 mM, and the single strand formation treatment by heat treatment of DNA is carried out, usually at 85°C or higher, preferably at 90°C or higher, and the upper limit usually at 100°C or lower, preferably at 95°C or lower, and usually for 30 seconds to 30 minutes, preferably for 1 minute to 5 minutes (to be used as (I)-C2 solution 1).

Then, the obtained above "(I)-C2 solution 1" is incubated under the lower limit usually at 15°C or higher, preferably at 30°C or higher, and the upper limit usually at 60°C or lower, preferably at 45°C or lower, and for 5 minutes to 480 minutes, preferably for 10 minutes to 60 minutes (referred to as (I)-C2 solution 2).

After that, using the above "(I)-C2 solution 2" instead of the above solution 1, and according to the method described in (I)-A, the single-stranded DNA in the "(I)-C2 solution 2" is contacted with the peroxide pertaining to the present invention.

### (II)-C1. The case where the alkali treatment of DNA consisting of 2 or more strands is carried out in the presence of the polyvalent metal oxides pertaining to the present invention and the peroxide pertaining to the present invention

For example, to a solution including 50 ng to 200 ng of the above DNA consisting of 2 or more strands, an aqueous solution including polyvalent metal oxides pertaining to the present invention and an aqueous solution containing peroxide pertaining to the present invention are added and mixed so that the final concentration of the polyvalent metal oxides pertaining to the present invention after mixing will be 100 mM to 3000 mM, preferably 200 mM to 2000 mM, and also, the final concentration of the peroxide pertaining to the present invention after mixing will be 10 mM to 200 mM, preferably 50 mM to 150 mM, and by adding 20 mol to 200 mol, preferably 50 mol to 100 mol alkali solution, the pH of the solution including DNA is made pH 10 to pH 14, preferably pH 12 to pH 14 (referred to as (II)-C1 solution 1).

Subsequently, the above "(II)-C1 solution 1" is incubated under the lower limit usually at 30°C or higher, preferably at 50°C or higher and the upper limit usually at 100°C or lower, preferably at 90°C or lower, most preferably at 70°C or lower, for 30 minutes to 480 minutes, preferably for 120 minutes to 300 minutes, and then the single-stranded DNA in the "(II)-C1 solution 1" is contacted with the polyvalent metal oxides pertaining to the present invention and the peroxide pertaining to the present invention

### (II)-C2. The case where the heat treatment of DNA consisting of 2 or more strands is carried out in the presence of the polyvalent metal oxides pertaining to the present invention and the peroxide pertaining to the present invention

For example, to a solution including 50 ng to 200 ng of the above DNA consisting of 2 or more strands, an aqueous solution including polyvalent metal oxides pertaining to the present invention and an aqueous solution containing peroxide pertaining to the present invention are added and mixed so that the final concentration of the polyvalent metal oxides pertaining to the present invention after mixing will be 100 mM to 3000 mM, preferably 200 mM to 2000 mM, and also, the final concentration of the peroxide pertaining to the present invention after mixing will be 10 mM to 200 mM, preferably 50 mM to 150 mM, and the single strand formation treatment by heat treatment of DNA is carried out, usually at 85°C or higher, preferably at 90°C or higher, and the upper limit usually at 100°C or lower, preferably at 95°C or lower, and usually for 30 seconds to 30 minutes, preferably for 1 minute to 5 minutes (referred to as (II)-C2 solution 1).

After that, the above "(II)-C2 solution 1" is incubated under the lower limit usually at 30°C or higher, preferably at 50°C or higher, the upper limit usually at 100°C or lower, preferably at 90°C or lower, most preferably at 70°C or lower, and for 30 minutes to 480 minutes, preferably for 120 minutes to 300 minutes, and the single-stranded DNA in the "(II)-C2 solution 1" is contacted with the polyvalent metal oxides pertaining to the present invention and the peroxide pertaining to the present invention.

Among specific examples of the above (A) to (C), the method of (B) and (C) are preferable, and the method of (B) is particularly preferable.

It should be noted that the present inventors have carried out an oxidation reaction of DNA extracted from living organism according to the method described in the above-mentioned known oxidation method (Non-Patent Literature 3, Patent Literature 2). However, in said method, the decomposition reaction of said DNA had been occurred, and detection of the objective hmC could not be performed. That is, the DNA extracted from living organism, in other words, the unmodified DNA cannot be used in the above-mentioned known oxidation reaction, the base modified with LNA (Locked Nucleic Acid) and BNA (Bridged Nucleic Acid) which have a nuclease-resistant cross-linked structure in the molecule has to be used.

On the other hand, it turned out that, according to the oxidation treatment using polyvalent metal oxides pertaining to the present invention and peroxide pertaining to the present invention, detection of hmC can be performed without being degraded even when the naturally occurring DNA is subjected to the oxidation reaction (see Example 5 to be described later).

In addition, when the above-mentioned known oxidation method is used, it is believed that the hmC is changed into thymine derivative (Non-Patent Literature 3, Patent Literature 2). On the other hand, when the method of the present invention is used, it was supposed that the hmC would be changed into cytosine derivative by oxidation.

That is, after carrying out the DNA oxidation step pertaining to the present invention to the DNA including a recognition sequence (one of the nucleic acids constituting a recognition sequence has cytosine or cytosine derivative) of restriction enzyme EcoRV, when restriction enzyme treatment using EcoRV was carried out, the recognition sequence of restriction enzyme EcoRV was cleaved by the restriction enzyme EcoRV. This suggests that when the method of the present invention is used, the hmC is changed into cytosine derivative by oxidation, in other words, the oxidation mechanism of hmC by the method of the present invention is different from oxidation reaction mechanism of hmC by the above-mentioned known oxidation method.

It should be noted that, it is characterized in that, in the method of the present invention, the DNA oxidation step [step (1)] is carried out using single-stranded DNA, that is, the polyvalent metal oxides pertaining to the present invention and the peroxide pertaining to the present invention are contacted with the single-stranded DNA. On the other hand, in the step after the DNA oxidation step [step (1)], either single-stranded DNA or double-stranded DNA can be used.

### (2) DNA purification step

After the DNA oxidation step pertaining to the present invention, the DNA (single-stranded DNA or double-stranded DNA) obtained by the DNA oxidation step may be purified by a purification method to be used usually in this field. Such a purification method is not specifically limited as long as it can purify the DNA, and, for example, alcohol precipitation method and column purification method are included.

More specifically, when performing a column purification method, it may be carried out for example as follows. That is, to the reaction solution obtained by the DNA oxidation step pertaining to the present invention, 300 µL to 500 µL of 1 mM to 100 mM Tris-HCl buffer solution (pH 5.5 to pH 7.5, preferably pH 6 to pH 7) containing 2 M to 6 M guanidinium hydrochloride is added and mixed, then the mixture is filled in EconoSpin (manufactured by Gene Design Inc.) and centrifuged at 10000 x g for 30 seconds to 5 minutes, preferably for 1 minute to 3 minutes to remove the flow-through liquid. After that, 400 µL to 750 µL of aqueous 80% ethanol containing 5 mM to 50 mM Tris-HCl (produced by Wako Pure Chemical Industries, Ltd.) aqueous solution (pH 6 to pH 8, preferably pH 7 to pH 7.5) is filled in EconoSpin and centrifuged at 10000 x g for 30 seconds to 5 minutes, preferably for 1 minute to 3 minutes to remove the flow-through liquid. After that, 20 µL to 100 µL, preferably 25 µL to 50 µL of 1 mM to 50 mM Tris-HCl (pH 8 to pH 9.5, preferably pH 8.5 to pH 9) is filled in EconoSpin and centrifuged at 10000 x g for 30 seconds to 5 minutes, preferably for 1 minute to 3 minutes to recover the flow-through liquid, thereby purified DNA can be obtained.

In addition, in order to perform purification by alcohol precipitation method, the purification may be carried out, for example, as follows. That is, to 10 µL of the reaction solution obtained by the DNA oxidation step pertaining to the present invention, usually 40 µL to 110 µL of alcohol and 30 µL to 100 µL of buffer solution are added, then subjected to centrifugal separation. After the centrifugal separation, the supernatant is removed, and by washing with alcohol, purified DNA is obtained. On the occasion of adding the above alcohol and buffer solution, in order to facilitate the removal of the supernatant after separation, 0.1 µL to 1 µL of coprecipitating agent or glycogen may be added to 10 µL of the solution including above DNA. The above alcohol includes ethanol, isopropanol, butanol and the like, and, isopropanol is particularly preferable. The bove buffer solution includes, for example, Good's buffer solution such as MES, HEPES, phosphate buffer solution, Tris buffer solution, glycine buffer solution, borate buffer solution, sodium bicarbonate buffer solution and the like, and among these, Good's buffer solution such as MES, HEPES, Tris buffer solution and the like are preferable, and Tris buffer solution is particularly preferable. The pH of these buffer solutions are usually pH 7 to pH 8, preferably pH 7 to pH 7.5, and the concentration of buffering agent in the buffer solution is usually 0.1 mol/L to 5 mol/L, preferably in the range from 0.1 mol/L to 2 mol/L. The above centrifugal separation is not particularly limited as long as it is the aspect to be done usually in this field, but usually it is performed at 10,000 x g to 22,000 x g for 10 minutes to 30 minutes.

### (3) DNA amplification step [step (2)]

The DNA amplification step is a step in which the DNA obtained in the DNA oxidation step pertaining to the present invention, or if needed, the DNA obtained in the further DNA purification step is subjected to the amplification treatment.

The "specific region of single-stranded DNA" pertaining to the present invention is a detection target region for determining the presence or absence of hmC in the determination step pertaining to the present invention, and it may be either a part or the entire length of the single-stranded DNA performed the treatment of the DNA oxidation step pertaining to the present invention.

In other words, in the case where the "specific region of single-stranded DNA" is the entire length of the single-stranded DNA performed the DNA oxidation step pertaining to the present invention, the entire length of said single-stranded DNA is the detection target region; and in the case where the "specific region of single-stranded DNA" is a part of the single-stranded DNA performed the DNA oxidation step pertaining to the present invention, a part of said single-stranded DNA is the detection target region.

In addition, in the DNA oxidation step pertaining to the present invention, the DNA to be subjected to the amplification treatment is a specific region of single-stranded DNA, however, when a single-stranded DNA and a complementary strand of said single-stranded DNA are present, not only the specific region of said single-stranded DNA but said "corresponding region of the complementary strand" may be subjected to the DNA amplification step (amplification treatment).

Here, the "corresponding region of the complementary strand" is a region (a sequence complementary to the sequence of the "specific region of single-stranded DNA") that binds complementarily to the "specific region of single-stranded DNA", it may be either the entire length or a part of the complementary strand of said single-stranded DNA.

In addition, the "complementary strand of single-stranded DNA" is a strand having a region (a sequence complementary to the sequence of the "specific region of single-stranded DNA") that binds complementarily to the "specific region of single-stranded DNA", and usually is the one having a sequence complementary to the sequence of the single-stranded DNA.

As described above, in the DNA amplification step pertaining to the present invention, in the case where said "specific region of single-stranded DNA" is to be subjected to the amplification treatment, the detection target region to determine the presence or absence of hmC in the determination step pertaining to the present invention is said "specific region of single-stranded DNA", and in the case where said "specific region of single-stranded DNA" and said "corresponding region of the complementary strand" are subjected to the amplification treatment, the detection target region to determine the presence or absence of hmC in the determination step pertaining to the present invention is not only said "specific region of single-stranded DNA", but two regions of this and said "corresponding region of the complementary strand".

The amplification treatment may be any method capable of amplifying DNA known per se, for example, amplification reaction method by DNA polymerase of polymerase chain reaction (PCR), LAMP method, isothermal gene amplification reaction method and the like are included. The amplification treatment using these known methods may be carried out according to the method known per se.

Among the above methods, in the case of using PCR, the sequence complementary to the nucleotide sequence of the 5'-terminal side and the sequence complementary to the nucleotide sequence of the 3'-terminal side of the "specific region of single-stranded DNA", the sequence complementary to the nucleotide sequence of the 5'-terminal side of the "corresponding region of the complementary strand", and/or the sequence complementary to the nucleotide sequence of the 3'-terminal side of the "corresponding region of the complementary strand" may be used as primer sequences (to be described later); in addition, an adapter sequence (to be described later) may be added to the 5'-or/and 3'-terminal of the "specific region of single-stranded DNA", and/or an adapter sequence may be added to the 5'- and/or 3'-terminal of the "corresponding region of the complementary strand". It should be noted that, in the case of performing amplification using the adapter, the amplification may be carried out, for example, according to the method disclosed in WO2009/044782.

The method for performing amplification treatment by means of the PCR includes, for example, the methods as described below.

### (Method-1)

A method for performing the PCR using a Forward primer (to be described later) containing a sequence complementary to the nucleotide sequence of the 5'-terminal side of the "specific region of single-stranded DNA" and a Reverse primer (to be described later) containing a sequence complementary to the nucleotide sequence of the 5'-terminal side of the "corresponding region of the complementary strand".

### (Method-2)

A method for performing the PCR, wherein the 3' adapter (to be described later) is added to the 5'-terminal side of the "specific region of single-stranded DNA" and the "corresponding region of the complementary strand", and 5' adapter (to be described later) is added to the 3'-terminal side of the "Specific region of single-stranded DNA" and the "corresponding region of the complementary strand", respectively, and using a Forward primer (to be described later) containing a sequence complementary to the 3' adapter in the entire or a part of the sequence and using a Reverse primer (to be described later) containing a sequence complementary to the 3' adapter in the entire or a part of the sequence, the PCR is carried out.

### (Method-3-1)

A method for performing the PCR, wherein the 3' adapter (to be described later) is added to the 5'-terminal side of the "specific region of single-stranded DNA", and using a Forward primer (to be described later) containing a sequence complementary to the 3' adapter in the entire or a part of the sequence, and using a Reverse primer (to be described later) containing a sequence complementary to the nucleotide sequence of the 5'-terminal side of the "corresponding region of the complementary strand", the PCR is carried out.

### (Method-3-2)

A method for performing the PCR, wherein the 3' adapter (to be described later) is added to the 5'-terminal side of the "corresponding region of the complementary strand", and using a Reverse primer (to be described later) containing a sequence complementary to the 3' adapter in the entire or a part of the sequence, and using a Forward primer (to be described later) containing a sequence complementary to the nucleotide sequence of the 5'-terminal side of the "Specific region of single-stranded DNA", the PCR is carried out.

### (Method-4)

A method for performing the PCR, wherein using a Forward primer (to be described later) containing a sequence complementary to the nucleotide sequence of the 5'-terminal side of the "specific region of single-stranded DNA" and using a Reverse primer (to be described later) containing a sequence complementary to the nucleotide sequence of the 5'-terminal side of the "corresponding region of the complementary strand" in the complementary strand generated when the PCR is carried out for 1 cycle, the PCR is carried out.

### (Method-5)

A method for performing the PCR, wherein the 3' adapter (to be described later) is added to the 5'-terminal side, and the 5' adapter (to be described later) is added to the 3'-terminal side of the "specific region of single-stranded DNA", respectively, and using a Forward primer (to be described later) containing a sequence complementary to the 3' adapter (to be described later) in the entire or a part of the sequence, and using a 5' primer (to be described later) containing a sequence of the 5' adapter in the entire or a part of the sequence, the PCR is carried out.

### (Method-6)

A method for performing the PCR, wherein the 5' adapter (to be described later) is added to the 3'-terminal side of the "specific region of single-stranded DNA" in a single-stranded DNA, and using a Forward primer (to be described later) containing a sequence complementary to the nucleotide sequence of the 5'-terminal side of the "Specific region of single-stranded DNA" in the entire or a part of the sequence, and using a 5' primer (to be described later) containing a sequence of the 5' adapter in the entire or a part of the sequence, the PCR is carried out.

### (Method-7)

A method for performing the PCR using a Forward primer (to be described later) containing a sequence complementary to the nucleotide sequence of the 5'-terminal side of the "specific region of single-stranded DNA".

The PCR method may be carried out according to a method well-known per se, for example, the methods described in Nucleic Acids Research, 1991, Vol. 19, 3749; Biotechniques, 1994, Vol. 16, 1134-1137.

The method will be specifically described below.

That is, to a 100 pg to 50 ng of the DNA performed the DNA oxidation step, if needed, DNA purification step, usually 0.1 pmol to 0.5 pmol, preferably 0.2 pmol to 0.3 pmol of a Forward primer (to be described later) and a Reverse primer (to be described later), respectively; usually 0.1 units to 5 units, preferably 0.5 units to 2.5 units of DNA polymerase (to be described later); and usually 50 µmol to 500 µmol, preferably 100 µmol to 300 µmol of 4 kinds of mixed deoxyribonucleotide triphosphates (dNTPs) are added, and in a 10 µL to 100 µL total volume of solution including 1 µL to 10 µL of a buffer solution such as Tricine buffer solution, Tris-HCl buffer solution, Universal buffer solution, for example, after heating at 90°C to 98°C for 10 seconds to 5 minutes, by setting the reactions "at 90°C to 98°C for 10 seconds to 1 minute, at 45°C to 65°C for 10 seconds to 1 minute, at 68°C to 72°C for 10 seconds to 2 minutes" as 1 cycle, 15 cycles to 40 cycles are carried out, and thus the PCR may be carried out at 68°C to 72°C for 30 seconds to 5 minutes,.

As to the Forward primer, Reverse primer and 5 'primer in the above PCR, description is described below. These primers may be used by preparing appropriately according to a method known per se.

The Forward primer is the one which contains a sequence complementary to the nucleotide sequence of the 5'-terminal side of the "specific region of single-stranded DNA", or the one which has a sequence complementary to the 3' adapter in the case of the method of adding 3' adapter on to the 5'-terminal of the "specific region of single-stranded DNA" (the above methods 2, 3-1, and 5), and the number of nucleotides thereof is usually 15 to 35, preferably 18 to 30, more preferably 20 to 28.

The Reverse primer is the one which contains a sequence complementary to the nucleotide sequence of the 5'-terminal side of the "corresponding region of the complementary strand", or the one which contains a sequence complementary to the 3' adapter in the case of the method for adding 3' adapter on to the 5'-terminal of the "corresponding region of the complementary strand" (the above methods 2, and 3-2), and the number of nucleotides thereof is usually 15 to 35, preferably 18 to 30, more preferably 20 to 28.

The 5' primer is the one which contains the sequence of 5' adapter in the entire or a part of the sequence, and the number of nucleotides thereof is usually 15 to 35, preferably 18 to 30, more preferably 20 to 28.

The above Forward primer, Reverse primer, and 5' primer (hereinafter, abbreviated as primer) are desirable to be a sequence which satisfies that hmC is not included in the primer and the DNA sequence to be bound with the primer, that the primer dimer is not formed just like the primers used in the usual PCR, and that the primer does not bind to the sequences other than the above complementary sequence.

The primer sequence is a sequence of 5' side and/or 3' side of the specific region of single-stranded DNA and/or the corresponding region of the complementary strand. The number of nucleotides thereof is usually 12 to 30, preferably 15 to 25, more preferably 18 to 22.

The above 5' adapter is described below.

As to the 5' adapter, it may be any sequence as long as the hydroxy group of the 3' side is treated so as not to react with the phosphate group, and the sequence is known DNA; and, the one which consists of a sequence that is not present in the DNA pertaining to the present invention is preferable. The number of nucleotides thereof is usually 12 to 30, preferably 15 to 25, more preferably 18 to 22.

The treatment which makes a hydroxy group of the 3' side not to react with the phosphate group may be the method as long as the method is carried out usually in this field, and for example, dehydroxylation of the 3'-terminal, and the treatment of coupling the biotin or the like to the hydroxy group at the 3'-terminal, etc. are included, however, among them, the dehydroxylation of the 3'-terminal is preferable. The one which was dehydroxylated at the 3'-terminal is able to be extracted easily, because it dissolves in an aqueous solution during extraction with a mixed solution of phenol/chloroform/isoamyl alcohol as a purification method.

As the method of adding the 3' adapter to 5'-terminal of the "specific region of single-stranded DNA" and/or the "corresponding region of the complementary strand", the 3' adapter pertaining to the present invention may be added to the 5'-terminal of the target DNA ("specific region of single-stranded DNA" and/or "corresponding region of the complementary strand") by a method known per se which is usually used in this field, and although it can be carried out using a commercially available kit, it may also be carried out, for example, according to the method described in Nucleic Acids Research, 1988, Vol. 16, No. 5, 1999-2014; Nucleic Acids Research, 1988, Vol. 16, No. 1, 265-277. Specifically, to the "specific region of single-stranded DNA" and/or the "corresponding region of the complementary strand", usually 10 pmol to 100 pmol, preferably 10 pmol to 50 pmol of the 3' adapter, and 1 unit to 50 units, preferably 5 units to 15 units of single-stranded DNA ligase are allowed to react at usually 50°C to 70°C, preferably at 50°C to 60°C, for usually 30 minutes to 90 minutes, preferably for 30 minutes to 90 minutes, in a buffer solution such as HEPES, Tris-HCl buffer, and MOPS. Thereby, a 3' adapter-added "specific region of single-stranded DNA" and/or a 3' adapter-added "corresponding region of the complementary strand" can be obtained. In said reaction, coenzyme such as ATP (adenosine triphosphate); reducing agent such as DTT (dithiothreitol); and reagents such as magnesium chloride, BSA, manganese chloride, which are commonly used at the time of such ligation, may be added, and the concentration and usage of these reagents are selected appropriately from the range that is usually used in this field.

In the method for adding the 3' adapter to the "specific region of single-stranded DNA" and/or the "corresponding region of the complementary strand" pertaining to the present invention, it is preferable to treat so as not to react with the hydroxy group of the 3'-terminal side of the "specific region of single-stranded DNA" and/or the "corresponding region of the complementary strand" by performing the above DNA purification step in advance.

The treatment thereof may be carried out according to the method usually performed in this field, there is included, for example, removal of residues by purification and dephosphorylation treatment by dephosphorylation enzyme and the like. Specifically, when the "specific region of single-stranded DNA" and/or the "corresponding region of the complementary strand" pertaining to the present invention is 100 bases or more, the above residues are removed by using a filter or a column of silica gel membrane and the like to be used usually in this field. In addition, it is also possible to remove the residues using a commercially available purification kit. When the "specific region of single-stranded DNA" and/or the "corresponding region of the complementary strand" pertaining to the present invention is 100 bases or less, due to the difficulty in removing the residues by the above filter and column, the removal of the residues is performed by carrying out dephosphorylation treatment for the residues. The dephosphorylation enzyme to be used here includes the same dephosphorylation enzyme as used for the dephosphorylation treatment performed prior to the above step 1 in the description of the above step 1, and preferred one, and deactivation · removal method are also the same.

After the 3' adapter is added to the 5'-terminal of the "specific region of single-stranded DNA" and/or the "corresponding region of the complementary strand", the added single-stranded DNA ligase, etc. are preferable to be deactivated, and the method may be carried out according to the method known per se depending on the enzyme to be used, and for example, it may be performed by heat treatment usually at 90°C to 100°C, preferably at 90°C to 95°C, usually for 5 minutes to 15 minutes, preferably for 5 minutes to 10 minutes. In addition, after enzyme deactivation, it is preferable to purify the 3' adapter added "specific region of single-stranded DNA" and/or "corresponding region of the complementary strand" by the purification method to be used usually in this field, for example, by the method of extraction with a mixed solution of phenol/chloroform/isoamyl alcohol, alcohol precipitation, column purification, and filtration through filter, and the like.

It should be noted that, the 3' adapter may be any sequence as long as the phosphate group of the 5' side is treated so as not to react with the hydroxy group and the sequence thereof is known DNA; and, the one which consists of a sequence that is not present in the "specific region of single-stranded DNA" and/or the "corresponding region of the complementary strand" pertaining to the present invention is preferable. The number of nucleotides thereof is usually 12 to 30, preferably 15 to 25, more preferably 18 to 22, and the specific addition method may follow the above-descried addition method for 5' adapter

The above adapter sequence is a sequence which is added to the 5'-terminal and/or 3'-terminal of the specific region of single-stranded DNA and/or the corresponding region of the complementary strand.

The number of nucleotides thereof is usually 12 to 30, preferably 15 to 25, and more preferably 18 to 22.

It should be noted that, in the objective amplification product (to be described later), in addition to the specific region of single-stranded DNA and/or the corresponding region of the complementary strand, an amplification product, in which the above adapter sequence is treated by amplification, is also included.

The DNA polymerase in the above PCR may be any DNA polymerase which is usually used in this field, there is included, for example, Taq DNA Polymerase such as Gene Taq (produced by Nippon Gene Co., Ltd.), and KOD DNA Polymerase, and the like.

The above dNTPs is not specifically limited as long as it is a mixture of 4 kinds of deoxyribonucleotide triphosphates (dATP, dCTP, dGTP, dTTP) commonly to be used in this field.

By the DNA amplification step pertaining to the present invention, the objective amplification product is obtained.

The objective amplification product includes, (i) the one which includes the entire length of the specific region of single-stranded DNA, (ii) the one which includes the entire length of the corresponding region of the complementary strand, (iii) the one which includes the entire length of the specific region of single-stranded DNA and the one which includes the entire length of the corresponding region of the complementary strand, or (iv) the one which includes further the adapter sequence in addition to these (hereinafter, (i) to (iv) are may be abbreviated collectively to "the objective amplification product pertaining to the present invention).

Therefore, the detection target region for determining the presence or absence of hmC in the determination step pertaining to the present invention may differ depending on the objective amplification product.

That is, in the case of the above (i) or (ii), the detection target region is the specific region of single-stranded DNA, and in the case of (iii), it is 2 areas of the specific region of single-stranded DNA and the corresponding region of the complementary strand thereof.

In other words, depending on the type (two regions of the specific region of single-stranded DNA or the corresponding region of the strand complementary to the aforementioned specific region) of detection target region to be desired and the amplification treatment method, the type [the amplification products of the above (i) to (iii)] of the amplification product, the type (two regions of the specific region of single-stranded DNA or the corresponding region of the strand complementary to the above specific region) of DNA in the DNA amplification step, and the type (presence or absence of complementary strand) of DNA in the DNA oxidation step, may be selected appropriately and determined.

These relationship is shown in Table 1 below.

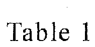

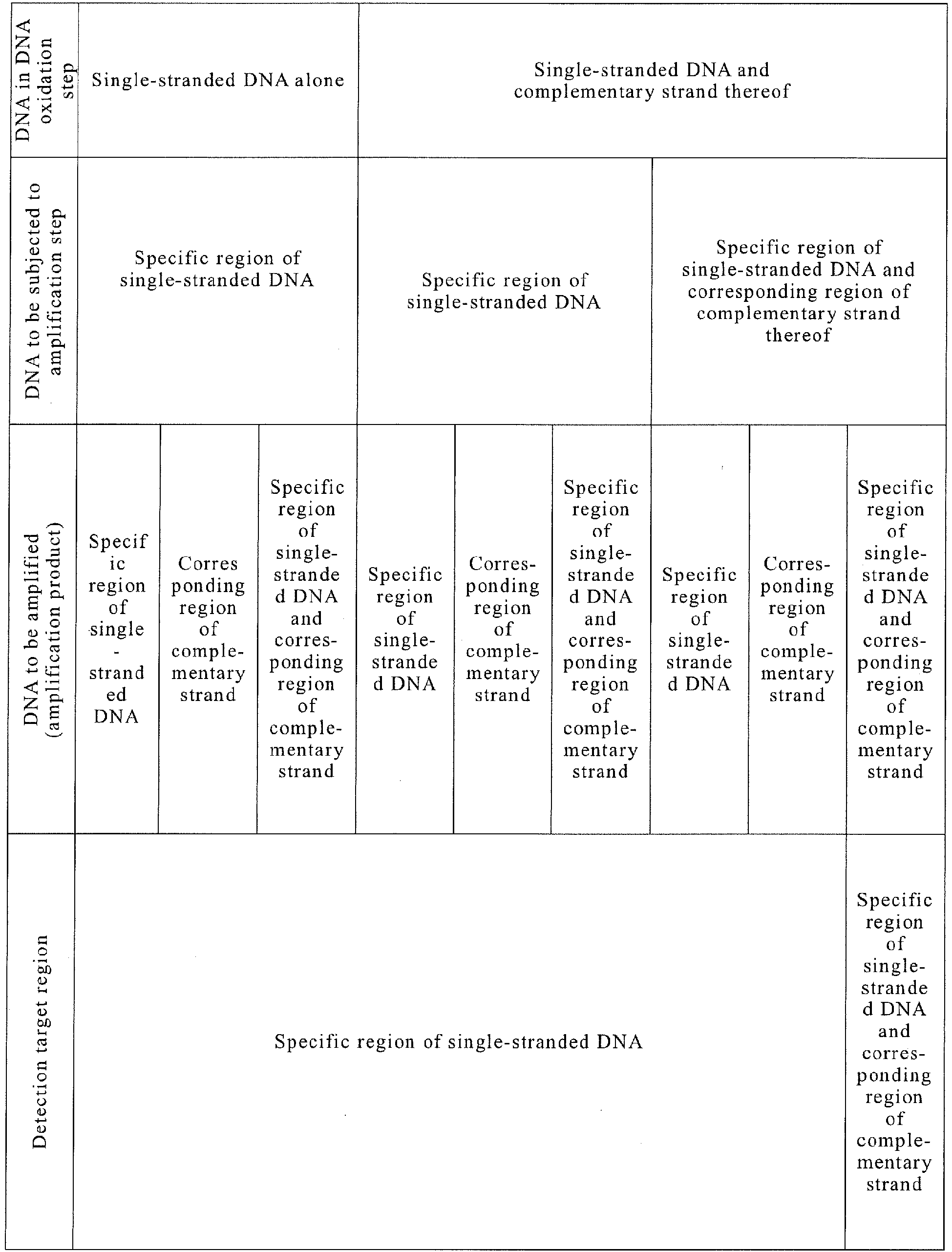

It should be noted that, in the amplification treatment using the above PCR, in the case where the sequence complementary to the nucleotide sequence of 3'-and/or 5'-terminal side of the "specific region of single-stranded DNA" and/or the "corresponding region of the complementary strand" is used as a primer sequence, because said primer sequence is a part of the specific region or the corresponding region, the primer sequence is to be included in the objective amplification product as well as in the detection target region. On the other hand, in the case where the adapter sequence is added to the 3'- and/or 5'-terminal of the "specific region of single-stranded DNA" and/or the "corresponding region of the complementary strand", said adapter sequence is included in the objective amplification product, however, because said adapter sequence is not a part of the specific region or the corresponding region, said adapter sequence is not included in the detection target region.

In Fig. 2, the relationship among the objective amplification product pertaining to the present invention, the specific region of single-stranded DNA, corresponding region of the complementary strand, detection target region, and primer sequence and adapter sequence, is shown.

The DNA amplification step may be carried out, for example, as follows.

For example, the DNA (single-stranded DNA or the single-strand DNA and the complementary strand thereof) obtained by the DNA oxidation step [step (1)], and if needed by the DNA purification step is subjected to the PCR as a template, thereby an objective amplification product pertaining to the present invention is obtained.

That is, to a 100 pg to 50 ng of the DNA performed the DNA oxidation step, if needed, DNA purification step, usually each 0.1 pmol to 0.5 pmol, preferably 0.2 pmol to 0.3 pmol of Forward primer and Reverse primer, usually 0.1 units to 5 units, preferably 0.5 units to 2.5 units of DNA polymerase, and usually 50 µmol to 500 µmol, preferably 100 µmol to 300 µmol of 4 kinds of mixed deoxyribonucleotide triphosphates (dNTPs) are added, and in a 10 µL to 100 µL total volume of solution including 1 µL to 10 µL of a buffer solution such as Tricine buffer solution, Tris-HCl buffer solution, Universal buffer solution, and, for example, after heating at 90°C to 98°C for 10 seconds to 5 minutes, by setting the reactions "at 90°C to 98°C for 10 seconds to 1 minute, at 45°C to 65°C for 10 seconds to 1 minute, at 68°C to 72°C for 10 seconds to 2 minutes" as 1 cycle, 15 cycles to 40 cycles are carried out, and by heating at 68°C to 72°C for 30 seconds to 5 minutes to perform the PCR, thereby the objective amplification product pertaining to the present invention performed the DNA oxidation step [step (1)] is obtained.

### (4) Amplification product detection step [step (3)]

Amplification product detection step is a step for detecting the presence or absence of objective amplification product pertaining to the present invention obtained by the DNA amplification step pertaining to the present invention.

The "objective amplification product" pertaining to the present invention is as described above.

That is, the objective amplification product pertaining to the present invention is the one obtained by the above DNA amplification step, and which includes (i) the one which includes the entire length of the specific region of single-stranded DNA, (ii) the one which includes the entire length of the corresponding region of the complementary strand, (iii) the one which includes the entire length of the Specific region of single-stranded DNA and the one which includes the entire length of the corresponding region of the complementary strand thereof, or (iv)the one which includes further the adapter sequence in addition to these.

On the other hand, the amplification products other than that described above, for example, the amplification products (amplification products not containing the entire length of the specific region and the corresponding region) including the primer dimer and only a portion of the sequence of the above specific region and the corresponding region, in the case of using the PCR as the amplification treatment, are excluded from the objective amplification products pertaining to the present invention.

The method for detecting presence or absence of the objective amplification product pertaining to the present invention includes the method for detecting the presence or absence of the amplification product to be used usually in this field, for example, electrophoresis method and chromatography method, and particularly preferably, agarose gel electrophoresis method.

For example, the agarose gel electrophoresis method includes the method disclosed in the paragraph of "Preparation of agarose gel and electromigration" in "Bio-experiment Illusted, ii Fundamental of Gene Analysis 2006, p.p. 54-58", etc. That is, a 0.6% to 2.0% agarose gel is set in the electrophoresis tank filled with 0.5 x TBE or 1 x TAE buffer, a solution including 1/10 to 1/5 amount of DNA subjected to amplification treatment in step (3) is mixed with loading buffer, and by applying a voltage of 100 V when 0.5 x TBE buffer is used, and 50 V when 1 x TAE buffer is used, respectively, the electrophoresis is carried out until the sample migrate about 1/2 to 1/3. After that, after staining the gel using a staining solution to be used usually in this field, for example, Gel Red, and the like, the presence or absence of the objective amplification product is detected by ultraviolet irradiation. That is, on the basis of the mobility after electrophoresis, whether it is the objective amplification product is determined. Specifically, for example, since the molecular weight and the mobility of nucleic acid is generally inversely proportional in a wide sense, molecular weight markers are electrophoresed as a measure of the mobility, and on the basis of the mobility to be expected from the molecular weight of the objective amplified product, the band obtained by electrophoresis is determined whether it is the objective amplification product.

### (5) Determination step [step (4)]

Determining step [step (4)] is a step in which, based on the result of the presence or absence of the objective amplification product detected in the amplification product detection step pertaining to the present invention, the presence or absence of hmC in the detection target region (the specific region of single-stranded DNA or the specific region of single-stranded DNA and the corresponding region of the complementary strand thereof) is determined.

In the case where hmC is included in the detection target region (the specific region of single-stranded DNA or the specific region of single-stranded DNA and the corresponding region of the complementary strand thereof), amplification reaction of the objective amplification product [(i) the one which includes the entire length of the specific region of single-stranded DNA, (ii) the one which includes the entire length of the corresponding region of the complementary strand, (iii) the one which includes the entire length of the specific region of single-stranded DNA and the one which includes the entire length of the corresponding region of the complementary strand, or (iv) the one which includes further the adapter sequence in addition to these] pertaining to the present invention in the DNA amplification step does not proceed, and the objective amplification product pertaining to the present invention is not detected in the amplification product detection step. This is considered that DNA polymerase cannot recognize an oxide of hmC in DNA (the specific region of single-stranded DNA and/or the corresponding region of the complementary strand) to be subjected to amplification treatment of the DNA amplification step, therefore, the extension reaction is stopped at the position of the oxide of hmC.

On the other hand, in the case where hmC is not included in the DNA (the specific region of single-stranded DNA and/or the corresponding region of the complementary strand) to be subjected to amplification treatment in the DNA amplification step, the amplification reaction of the objective amplification product pertaining to the present invention in the DNA amplification step proceeds, and the objective amplification product pertaining to the present invention is detected in the amplification product detection step.

That is, when the objective amplification product pertaining to the present invention is detected in the amplification product detection step, it is determined that hmC is not included in the detection target region (the specific region of single-stranded DNA or the specific region of single-stranded DNA and the corresponding region of the complementary strand thereof). On the other hand, when the objective amplification product pertaining to the present invention is not detected in the amplification product detection step, it is determined that hmC is included in the detection target region (the specific region of single-stranded DNA or the specific region of single-stranded DNA and the corresponding region of the strand complementary thereto).

### 5. Reagent kit of the present invention

The reagent kit of the present invention is the one to be used for performing the method for detecting hmC in DNA pertaining to the present invention as described above.

A reagent kit for detecting hmC in DNA pertaining to the present invention includes those comprising:
(i) a reagent including the polyvalent metal oxides pertaining to the present invention, and
(ii) a reagent including a peroxide pertaining to the present invention.

A preferable aspect and specific examples of these constituent features are as mentioned above.

Further, it is possible to make a kit of the present invention by adding reagents other than the reagents listed above. That is, such reagents may comprise, for example, the following a) to d), but are not limited thereto.
a) A column for nucleic acid purification (for example, EconoSpin, manufactured by Gene Design, Inc.)
b) Buffer solution to be used in DNA purification step (for example, chelating agent containing Tris-HCl buffer solution)
c) Reagents for the PCR (for example, one type or 2 or more types of primers, one type or 2 or more types of adapters)
d) Reagents for electrophoresis (for example, agarose gel, loading buffer solution, Gel Red, ethidium bromide staining reagent)

In addition, in said kit, a manual for performing the detecting method of hmC in DNA pertaining to the present invention as mentioned above may be included. Said "manual" means the instruction manual, the package insert, or brochure (leaflet), and the like of said kit, in which features, principles and operating procedures and the like in the method of the present invention are described substantially in text or by figures and tables and the like.

The reagents to be included in the reagent kit of the present invention are diluted by being mixed with DNA solution, alkali solution or the like at the time of use, and, the effective concentration at the time of use may be in the range of concentration of the above-mentioned polyvalent metal oxide pertaining to the present invention and in the range of concentrations of the aforementioned peroxide pertaining to the present invention.

Hereinafter, the present invention will be explained in more detail by referring to Experimental Example, Examples, and Comparative Examples and the like, however, the present invention is not limited thereto in any way.

### EXAMPLES

### Experimental Example 1: Amplification of template DNA

Using SEQ ID NO: 1 (region of 566 bp) in lambda DNA (produced by Nippon Gene Co., Ltd.) as a template DNA, amplification was carried out by the PCR method, and polynucleotides having cytosine, methylated cytosine (mC) or hydroxymethylated cytosine (hmC) were obtained.

That is, 3 kinds of reaction solution of total 50 µL were prepared by mixing the following (a) to (i), and after heating at 94°C for 30 seconds, by setting the reactions "at 94°C for 20 seconds, at 58°C for 20 seconds, at 72°C for 20 seconds" as 1 cycle, 25 cycles of PCR are carried out, and then heated at 72°C for 1 minute.
(a) an aqueous solution containing 1ng of lambda DNA (produced by Nippon Gene Co., Ltd.), 34.5 µL;
(b) 10 × Universal Buffer (produced by Nippon Gene Co., Ltd.), 5 µL;
(c) 10 mM dATP (produced by Wako Pure Chemical Industries, Ltd.), 1 µL;
(d) 10 mM dGTP (produced by Wako Pure Chemical Industries, Ltd.), 1 µL;
(e) 10 mM dTTP (produced by Wako Pure Chemical Industries, Ltd.), 1 µL;
(f) 5 µM Forward primer (SEQ ID NO: 2) solution (produced by Sigma-Aldrich Japan Co. LLC.), 3 µL;
(g) 5 µM Reverse primer (SEQ ID NO: 3) solution (produced by Sigma-Aldrich Japan Co. LLC.), 3 µL;
(h) 5 Units/µL Gene Taq (produced by Nippon Gene Co., Ltd.), 0.5 µL;
(i) 10 mM dCTP (produced by Wako Pure Chemical Industries, Ltd.) 1µL, 10 mM dmCTP (methylated dCTP) (produced by Thermo Fisher Scientific K.K.) 1 µL, or 10 mM dhmCTP (hydroxymethylated dCTP) (produced by Nippon Gene Co., Ltd.) 1 µL.

After that, each of the obtained PCR amplification product was electrophoresed on a 1.5% agarose gel (produced by Nippon Gene Co., Ltd.), and after staining with GelRed (produced by Wako Pure Chemical Industries, Ltd.), subjected to ultraviolet irradiation, and the "amplification product of 566 bp" by the PCR was cut out from the gel, and the "amplification product of 566 bp" was extracted from said gel by QIAquick Gel Extraction Kit (produced by QIAGEN, Inc.).

Further, for the 3 kinds of "amplification product of 566 bp", after measuring the absorbance of extraction liquid by a spectrophotometer, each extraction liquid was diluted with distilled water (produced by Otsuka Pharmaceutical Co., Ltd.) so as to make each extraction liquid to be 26 µL of diluted solution including 100 ng of the "amplification product of 566 bp", and the obtained 3 kinds of diluted solution were used as reaction solution 1.

It should be noted that the above SEQ ID NO: 1 to SEQ ID NO: 3 are the DNA having the following sequences:
SEQ ID NO: 1: The 566 bp region of lambda DNA to be amplified by the PCR, Genbank Accession No. J02459: 26604-27169 (Strand = Plus / Minus);
SEQ ID NO: 2: Forward primer, Genbank Accession No. J02459: 27144-27169, GCAACATGAATAACAGTGGGTTATCC;
SEQ ID NO: 3: Reverse primer, Genbank Accession No. J02459: 26604-26626, CAATGTCGGCTAATCGATTTGGC.

### Example 1 and 2: Detection of hmC in DNA

### (1) Single strand formation of double-stranded DNA obtained in Experimental Example 1

To a 26 µL of reaction solution 1 obtained in Experimental Example 1, 4 µL of 1 M sodium hydroxide was added to make the reaction solution pH 12 to pH 14, and the obtained solution was used as reaction solution 2.

### (2) Contact of single-stranded DNA and polyvalent metal oxides (hereinafter, it may be abbreviated as DNA oxidation step 1-1)

To the reaction solution 2 obtained in (1), 20 µL of 2 M aqueous solution of sodium tungstate (produced by Wako Pure Chemical Industries, Ltd.) (Example 1) or 20 µL of 2 M aqueous solution of potassium tungstate (produced by Wako Pure Chemical Industries, Ltd.) (Example 2) was added and mixed (50 µL in total), and incubated at 30°C for 10 minutes. The obtained solution was used as reaction solution 3.

### (3) Contact of single-stranded DNA and peroxide (hereinafter, it may be abbreviated as DNA oxidation step 1-2 or 1-2)

To the reaction solution 3 obtained in the above (2), 50 µL of 300 mM oxone (produced by Wako Pure Chemical Industries, Ltd.) was added and mixed (100 µL in total), then incubated at 60°C for 4 hours, and the obtained solution was used as reaction solution 4.

### (4) Column purification of DNA

To the reaction solution 4 obtained in the above (3), 500 µL of 20 mM Tris-HCl buffer solution (pH 6.6) containing 5.5 M guanidinium hydrochloride (produced by Wako Pure Chemical Industries, Ltd.) was added and mixed, and filled in EconoSpin (manufactured by Gene Design Co. Ltd.), and centrifuged at 10000 × g for 1 minute to remove the flow-through liquid. Then, after placing a 650 µL of 2 mM Tris-HCl buffer solution (pH 7.5) (produced by Gene Design Co. Ltd.) containing 80% ethanol (Wako Pure Chemical Industries, Ltd.) in the EconoSpin, centrifuged at 10000 × g for 1 minute to remove the flow-through liquid. Further, a 50 µL of 10 mM Tris-HCl (pH 9.0) (produced by Nippon Gene Co., Ltd.) was filled in EconoSpin, and centrifuged at 10000 × g for 1 minute, and the flow-through liquid was recovered to obtain purified DNA.

### (5) PCR amplification (DNA amplification step [step (2)])

Using the purified DNA to be included in the flow-through liquid recovered in the above (4) as a template, DNA (SEQ ID NO: 4 and complementary strand thereof) was amplified by the PCR method.

That is, the reaction solution of total 25 µL was prepared by mixing the following (a) to (g), and after heating at 94°C for 30 seconds, by setting the reactions "at 94°C for 20 seconds, at 58°C for 20 seconds, at 72°C for 20 seconds" as 1 cycle, for 25 cycles, then heated at 72°C for 1 minute; thus the PCR was carried out.
(a) flow through liquid including 2 ng equivalent DNA, recovered in (4), 1 µL;
(b) distilled water (produced by Otsuka Pharmaceutical Co., Ltd.), 16.3 µL;
(c) 10 × Universal Buffer (produced by Nippon Gene Co., Ltd.), 2.5 µL;
(d) 2.5 mM dNTPs (produced by Nippon Gene Co., Ltd.), 2 µL;
(e) 5 µM Forward primer (SEQ ID NO: 5) (produced by Sigma-Aldrich Japan Co. LLC.), 1.5 µL;
(f) 5 µM Reverse primer (SEQ ID NO: 6) (produced by Sigma-Aldrich Japan Co. LLC.), 1.5 µL;
(g) Gene Taq (5 Units/µL) (produced by Nippon Gene Co., Ltd.), 0.2 µL.

It should be noted that the above SEQ ID NO: 4 to SEQ ID NO: 6 are the DNA having the following sequences:
SEQ ID NO: 4: Amplified region of 177 bp of lambda DNA, Genbank Accession No. J02459: 26795-26971 (Strand = Plus / Minus);
SEQ ID NO: 5: Forward primer, Genbank Accession No. J02459: 26945-26971, GTTGGAGTTTAGTGTTATTGAAAGAGG;
SEQ ID NO: 6: Reverse primer, Genbank Accession No. J02459: 26795-26819, CCTACAAAACCAATTTTAACATTTC;

### (6) Detection of the amplified product by electrophoresis (Amplification product detection step [step (3)])

The PCR amplification product obtained in the above (5) was electrophoresed on a 2% agarose gel (produced by Nippon Gene Co., Ltd.), and after staining with GelRed (produced by Wako Pure Chemical Industries, Ltd.), the presence or absence of the objective amplification product (177 bp) was determined by ultraviolet irradiation and the results thereof are shown in Fig. 3.

Fig. 3 (A) shows the results in the case of using a sodium tungstate (Example 1), and Fig. 3 (B) shows the results in the case of using potassium tungstate (Example 2).

As is clear from Fig. 3, when the PCR is carried out for the DNA including cytosine or mC after oxidation treatment, a band of the objective amplification product (177 bp) was observed, however, in the PCR carried out for the DNA including hmC after oxidation treatment, a band of the objective amplification product (177 bp) was not observed.

### (7) Determination of the presence or absence of hmC (Determination step [step(4)])

In the case where hmC is not included in the DNA (in the case of using the DNA containing cytosine or mC), a band of the objective amplification product (177 bp) was detected by electrophoresis.

On the other hand, in the case where hmC is contained in the DNA, a band of the objective amplification product (177 bp) was not detected by electrophoresis.

That is, it is considered that when hmC is not included in the DNA, the polymerase recognizes cytosine or mC and the PCR proceeds, whereas when hmC is included in the DNA, the polymerase cannot recognize hmC and the PCR does not proceed.

Therefore, if there is the objective amplification product (177 bp), it can be determined that hmC is not included in the DNA of detection object, and if there is not the objective amplification product (177 bp), it can be determined that hmC is included in the DNA of detection object.

That is, it turned out that if the method of the present invention is used, the presence or absence of hmC in the specific region of DNA and the complementary strand thereof can be detected with high accuracy. It should be noted that the result is shown in Table 9 below together with other Examples and Comparative Examples.

### Example 3 and 4: Detection of hmC in DNA

The method of the present invention was carried out by the same manner as in Example 1 except for using 20 µL of 2 M aqueous tungstic acid solution (Example 3) or 20 µL of 2 M aqueous sodium molybdate solution (Example 4) as an aqueous solution containing polyvalent metal oxides in place of 20 µL of 2 M aqueous sodium tungstate solution, and except for carrying out the PCR for 15 cycles or 20 cycles. The obtained results are shown in Fig. 4 (A) and Fig. 4 (B), respectively.

As is clear from the results of Fig. 4, when tungstic acid or sodium molybdate was used as a polyvalent metal oxide, as with the case of using sodium tungstate (Example 1) and the case of using potassium tungstate (Example 2), when hmC was not included in the DNA, amplification by PCR proceeds, and a band of the objective amplification product (177 bp) was identified by electrophoresis.

In addition, when hmC was included in the DNA, amplification by PCR did not proceed, and a band of the objective amplification product (177 bp) was not identified by electrophoresis.

That is, it turned out that when tungstic acid or sodium molybdate which are "the polyvalent metal acid salt of a metal selected from group 6 of the periodic table" is used as a polyvalent metal oxide, also, the presence or absence of hmC in DNA can be detected with high accuracy. It should be noted that, the results were shown in Table 9 together with other Examples and Comparative Examples.

### Example 5: Detection of hmC in genomic DNA

By the method of the present invention, whether it is possible to detect the presence or absence of hmC in naturally-occurring genomic DNA was examined.

That is, using the intron region (SEQ ID NO: 7) between exon 1 and exon 2 of the gene of Epidermal Growth Factor Receptor (EGFR) (Journal of Biological Chemistry, 2011, Vol.286, 24685-24693), in which many hydroxymethylated cytosine is considered to exist in nerve cells of the brain, as a "specific region of single-stranded DNA", the presence or absence of hmC was examined.

Specifically, the method of the present invention was carried out by the same manner as carried out in Experimental Example 1, in place of 26 µL of reaction solution 1 including double-stranded DNA obtained by Example 1, and except for carrying out the PCR by setting the PCR cycle as 40 cycles under the following condition, using 100 ng of "specific region of single-stranded DNA and a complementary strand thereof" in the DNA derived from IMR-32 (Riken Cell Bank) that is human neuroblastoma,.

It should be noted that, as a control, using DNA derived from HEK293 (JCRB Cell Bank) that is human renal cell, DNA derived from HepG2 (JCRB Cell Bank) that is human hepatoma cell, or DNA derived from K562 (JCRB Cell Bank) that is human chronic myelogenous leukemia cell, respectively, experiments were carried out in the same manner as carried out for the DNA derived from the above IMR-32.

Condition of the PCR is as follows.

The method of the present invention was carried out by the same manner as carried out in Example 1 except that, using 26 µL of aqueous solution including 100 ng of the above "specific region of single-stranded DNA and complementary strand thereof", in addition, using 3 µL of 5 µM Forward primer (SEQ ID NO: 8) solution and 3 µL of 5 µM Reverse primer (SEQ ID NO: 9) solution, the PCR was carried out by setting the number of PCR cycle as 40 cycles.

As a result, when the DNA derived from IMR-32, in which many hmC was considered to exist, was used, amplification by PCR did not proceed, and the band of the objective amplification product (163 bp) was not identified by electrophoresis.

On the other hand, when HEK293-derived DNA, HepG2-derived DNA, and K562-derived DNA, which were used as control, were used, amplification by PCR proceeded, and the band of the objective amplification product (163 bp) was identified by electrophoresis.

That is, it turned out that according to the method of the present invention, even it is the DNA which is derived from naturally occurring nucleic acid, hmC in the DNA can be detected. The results were shown in Table 9 together with other Examples and Comparative Examples.

It should be noted that the above SEQ ID NO: 7 to SEQ ID NO: 9 are the DNA having the following sequences:
SEQ ID NO: 7: PCR amplified region (163 bp) of EGFR, Genbank Accession No. AY588246: 65448-65610;
SEQ ID NO: 8: Forward primer, Genbank Accession No. AY588246 :65448-65473,
GCTCCAGTGTAGACATACAATAGACC; SEQ ID NO: 9: Genbank Accession No. AY588246 :65590-65610, CTGCAGCTTCTTAAGCCATGG.

### Example 6: Detection of hmC in DNA, by single strand formation treatment by heat treatment and simultaneous oxidation of DNA

### (1) Single strand formation of double-stranded DNA

To a 26 µL of reaction solution 1 obtained in Experimental Example 1, 20 µL of 2 M aqueous sodium tungstate solution (produced by Wako Pure Chemical Industries, Ltd.) and 50 µL of 300 mM oxone (produced be Wako Pure Chemical Industries, Ltd.) were added and mixed.

Then, by incubating at 90°C for 3 minutes, single strand formation of amplification product of 566 bp obtained in Experimental Example 1 was carried out, and used it as a reaction solution 1.

### (2) Contact of single-stranded DNA with polyvalent metal oxides and peroxide (hereinafter, it may be abbreviated as DNA oxidation step)

The reaction solution 1 obtained in the above (1) was incubated at 60°C for 4 hours, and the obtained solution was used as reaction solution 2.

From the column purification of the DNA to the determination of the presence or absence of hmC, using the reaction solution 2 obtained in the above (2) instead of using reaction solution 4, experiment was carried out by the same manner as carried out in (4) to (7) of Example 1.

As a result, in same manner as Example 1 in which after the single-stranded DNA is subjected to contact with sodium tungstate, the single-stranded DNA is oxidized by being contacted with oxone, in the case of DNA in which hmC is not included, amplification by the PCR proceeded, and the band of objective amplification product (177 bp) was identified by electrophoresis. On the other hand, in the case of DNA which includes hmC, amplification by PCR did not proceed, and the band of objective amplification product (177 bp) was not identified by electrophoresis.

That is, it was confirmed that in the method of the present invention, the presence or absence of hmC in DNA can be detected even when the single-stranded DNA is contacted with the polyvalent metal oxides pertaining to the present invention and the peroxide pertaining to the present invention "simultaneously". It should be noted that, the results were shown in Table 9 together with other Examples and Comparative Examples.

### Comparative Example 1 and 2: Examination using sodium perchlorate or potassium permanganate

Detection of hmC was carried out by the same manner as in Example 1 except that DNA oxidation step 1-1 and 1-2 were carried out under the condition described below.

That is, the experiments were carried out using 20 µL of 100 mM or 10 mM potassium permanganate instead of 20 µL of 2 M sodium tungstate, and by setting the temperature condition of DNA oxidation step 1-1 and step 1-2 as:
(a) DNA oxidation step 1-1 is carried out at 30°C, and DNA oxidation step 1-2 is carried out at 60°C; or
(b) DNA oxidation step 1-1 and DNA oxidation step 1-2 are carried out on ice.

The differences from the experimental conditions of Example 1 are described in Table 2 together with the experimental conditions of Comparative Example 2.

It should be noted that the potassium permanganate is a "polyvalent metal oxide of a metal selected from metals in group 7 of the periodic table (hereinafter, it may be abbreviated as "polyvalent metal oxide of a metal in the group 7").

Detection of hmC was carried out by the same manner as in Example 1 except that DNA oxidation step 1-1 and step 1-2 were carried out under the condition described below. That is, the experiments were carried out by using 20 µL of 1 M or 100 mM sodium perchlorate instead of using 20 µL of 2 M sodium tungstate, and by setting the temperature condition of DNA oxidation step 1-1 and step 1-2 as:
(a) DNA oxidation step 1-1 is carried out at 30°C, and step 1-2 is carried out at 60°C; or
(b) DNA oxidation step 1-1 and step 1-2 are carried out on ice.

The differences from the experimental conditions of Example 1 are described in Table 2 together with the experimental conditions of Comparative Example 1.

It should be noted that the sodium perchlorate is a "peroxide of an element in group 17 of the periodic table (hereinafter, it may be abbreviated as "peroxide of an element in the group 17").

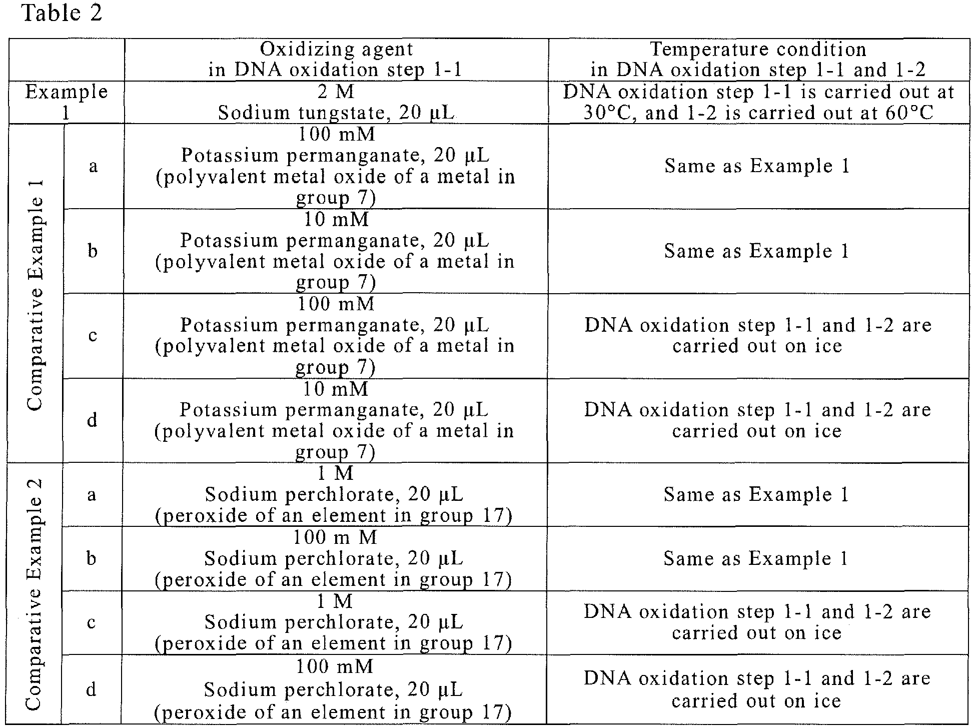

As a result described above, when potassium permanganate (Comparative Example 1) or sodium hypochlorite (Comparative Example 2) are used as an oxidizing agent, regardless of the temperature at reaction and the use concentration of oxidizing agent, the band of the objective amplification product (177 bp) could not be identified.

Further, even when the hmC does not exist, the band of the objective amplification product was not identified.

This was considered that due to strong oxidizing power of potassium permanganate (Comparative Example 1) or sodium hypochlorite (Comparative Example 2), DNA was degraded.

That is, it turned out that when the polyvalent oxides of metals in group 7 or a peroxide of an element in group 17 was used, hmC cannot be detected, in other words, when an oxidizing agent other than the polyvalent metal oxides pertaining to the present invention is used, hmC cannot be detected. It should be noted that, the results were shown in Table 9 below together with other Examples and Comparative Examples.

### Comparative Example 3 to 7: Examination using sodium periodate, 4-methylmorpholine N-oxide, 2-iodobenzene sulfonic acid, sodium orthovanadate, and copper chloride

Detection of hmC was carried out by the same manner as in Example 1 except that DNA oxidation step 1-1 and step 1-2 were carried out under the condition described below.

That is, the experiments were carried out by using 20 µL of 200 mM sodium periodate instead of 20 µL of 2 M sodium tungstate, and by setting the temperature condition of DNA oxidation step 1-1 and step 1-2 as:
(a) DNA oxidation step 1-1 is carried out at 30°C, and 1-2 is carried out at 60°C (hereinafter, it may be abbreviated as "Comparative Example 3-a"); or
(b) DNA oxidation step 1-1 and 1-2 are carried out on ice (hereinafter, it may be abbreviated as "Comparative Example 3-b") (Comparative Example 3).

The differences from the conditions of Example 1 were described in Table 3 together with the experimental conditions of Comparative Example 4 to 7.

It should be noted that the sodium periodate is a "oxide of an element in group 17".

Detection of hmC was carried out by the same manner as in Example 1 except that DNA oxidation step 1-1 and 1-2 were carried out under the condition described below.

That is, the experiments were carried out by using 20 µL of 2 M 4-methylmorpholine N-oxide (Comparative Example 4), 20 µL of 2 M 2-iodobenzene sulfonic acid (Comparative Example 5), 20 µL of 2 M sodium orthovanadate (Comparative Example 6), and 20 µL of 2 M copper chloride (Comparative Example 7), instead of 20 µL of 2 M sodium tungstate.

The differences from the conditions of Example 1 are described in Table 3 below together with the experimental conditions of Comparative Example 3.

It should be noted that the 4-methylmorpholine N-oxide (Comparative Example 4) and 20 uL of 2-iodobenzene sulfonic acid (Comparative Example 5) are oxidizing agent, and sodium orthovanadate (Comparative Example 6) is a "polyvalent metal oxides or polyvalent metal acid salt (hereinafter, it may be abbreviated as "polyvalent metal oxides of metals in group 5") of a metal selected from metals in group 5 of the periodic table", and 20 µL of copper chloride (Comparative Example 7) is a "compound (hereinafter, it may be abbreviated as "compound of the element in group 11") of element in group 11 of the periodic table".

**Table 3**

| | | Oxidizing agent in DNA oxidation step 1-1 | Temperature condition in DNA oxidation step 1-1 and 1-2 |
|---|---|---|---|
| Example 1 | | 2 M | DNA oxidation step 1-1 is carried out at 30°C, and 1-2 is carried out at 60°C |
| | | Sodium tungstate, 20 µL | |
| Comparative Example 3 | a | 200 mM Sodium periodate, 20 µL (oxide of an element in group 17) | Same as Example 1 |
| Comparative Example 3 | b | 200 mM | DNA oxidation step 1-1 and 1-2 are carried out on ice |
| | | Sodium periodate, 20 µL (oxide of an element in group 17) | |
| Comparative Example 4 | | 2 M | Same as Example 1 |
| | | 4-methylmorpholine N-oxide, 20 µL (oxidizing agent) | |
| Comparative Example 5 | | 2 M | Same as Example 1 |
| | | 2-iodobenzene sulfonic acid, 20 µL (oxidizing agent) | |
| Comparative Example 6 | | 2 M | Same as Example 1 |
| | | Sodium orthovanadate, 20 µL (polyvalent metal oxide of a metal in group 5) | |
| Comparative Example 7 | | 2 M | Same as Example 1 |
| | | Copper chloride, 20 µL (compound of an element in group 11) | |

As a result, in the "Comparative Example 3-a", regardless of the presence or absence of hmC in DNA, a band of the objective amplification product (177 bp) was not identified by electrophoresis.

The reason for this was considered that DNA was degraded by a strong oxidizing power of sodium periodate.

On the other hand, in the result of "Comparative Example 3-b", in the result of using 4-methyl morpholine N-oxide (Comparative Example 4), in the result of using 2-iodobenzene sulfonic acid (Comparative Example 5), in the result of using sodium orthovanadate (Comparative Example 6), or in the result of using copper chloride (Comparative Example 7), regardless of the presence or absence of hmC in DNA, a band of the objective amplification product (177 bp) was identified by electrophoresis.

That is, it was considered that, by the reasons that when "oxides of elements in group 17" (Comparative Example 3-b), "polyvalent metal oxides of metals in group 5" (Comparative Example 6), "compound of the element in group 11" (Comparative Example 7) were used, (i) oxidation of hmC proceeded in a reaction mechanism different from the case of using polyvalent metal oxides, or (ii) oxidation of hmC did not proceed completely, etc., the structure of hmC after oxidation reaction was different from the case of using polyvalent metal oxides, therefore, amplification by PCR proceeded even it was the oxidatively-treated DNA, and the band of the objective amplification product (177 bp) was identified by electrophoresis.

In addition, it was also considered that when 4-methylmorpholine N-oxide (Comparative Example 4) or 2-iodobenzene sulfonic acid (Comparative Example 5) was used, because the steric structure of 4-methylmorpholine N-oxide (Comparative Example 4) or 2-iodobenzene sulfonic acid (Comparative Example 5) is bulky, steric hindrance occurred, and because oxidation of hmC did not proceed completely, the structure of hmC after oxidation reaction was different from the case with polyvalent metal oxides, therefore, amplification of the oxidatively-treated DNA by PCR proceeded, and the band of the objective amplification product (177 bp) was identified by electrophoresis.

That is, it turned out that in the case of using 4-methylmorpholine N-oxide, 2-iodobenzene sulfonic acid, polyvalent metal oxides of the metals in group 5 or a compound of element in group 11, hmC cannot be detected, in other words, when an oxidizing agent other than the polyvalent metal oxides pertaining to the present invention is used, hmC cannot be detected. It should be noted that, the results were shown in Table 9 below together with other Examples and Comparative Examples.

### Comparative Example 8: Examination using hydrogen peroxide

Detection of hmC was carried out by the same manner as in Example 1 except that DNA oxidation step 1-1 and 1-2 were carried out under the condition described below.

That is, the experiments were carried out by using 30% hydrogen peroxide so as to provide 1% or 0.1% of a final volume concentration in the reaction solution containing single-stranded DNA instead of oxone, and by setting the temperature condition of DNA oxidation step 1-1 and 1-2 as:
(a) DNA oxidation step 1-1 is carried out at 30°C, and 1-2 is carried out at 50°C;
   or
(b) DNA oxidation step 1-1 and 1-2 are carried out on ice.

The differences from the experimental conditions of Example 1 are listed in Table 4 below.

**Table 4**

| | | Oxidizing agent in DNA oxidation step 1-1 | Temperature condition in DNA oxidation step 1-1 and 1-2 |
|---|---|---|---|
| Example 1 | | 300 mM | DNA oxidation step 1-1 is carried out at 30°C, and 1-2 is carried out at 60°C |
| | | Oxone, 50 µL | |
| Comparative Example 8 | a | A volume of 30% hydrogen peroxide which provides 1% final volume concentration in the reaction solution | DNA oxidation step 1-1 is carried out at 30°C, and 1-2 is carried out at 50°C |
| Comparative Example 8 | b | A volume of 30% hydrogen peroxide which provides 1% final volume concentration in the reaction solution | DNA oxidation step 1-1 and 1-2 are carried out on ice |
| Comparative Example 8 | c | A volume of 30% hydrogen peroxide which provides 0.1% final volume concentration in the reaction solution | DNA oxidation step 1-1 is carried out at 30°C, and 1-2 is carried out at 50°C |
| Comparative Example 8 | d | A volume of 30% hydrogen peroxide which provides 0.1% final volume concentration in the reaction solution | DNA oxidation step 1-1 and 1-2 are carried out on ice |

As a result, regardless of the presence or absence of hmC in DNA, also, regardless of the difference in the temperature condition and the concentration of hydrogen peroxide, amplification by PCR did not proceed, and a band of the objective amplification product (177 bp) was not identified by electrophoresis.

This cause was considered that to be due to a strong oxidizing power of hydrogen peroxide, DNA was degraded.

That is, it turned out that when a peroxide other than the peroxides pertaining to the present invention is used, hmC cannot be detected. It should be noted that, the results were shown in Table 9 below together with other Examples and Comparative Examples.

### Comparative Example 9: Examination of bringing single-stranded DNA into contact only with peroxide

Detection of hmC was carried out without carrying out DNA oxidation step 1-1, but by the same manner as in Example 1 except for carrying out under the condition described below.

That is, in the single strand formation of double-stranded DNA, the experiments were carried out by using 25 µL of the reaction solution 1 containing 100 ng of double-stranded DNA obtained in Experimental Example 1 instead of using 26 µL of the reaction solution 1 containing 100 ng of double-stranded DNA obtained in Experimental Example 1, and using 20 µL of 1 M NaOH instead of using 4 µL of 1 M NaOH.

The differences from the experimental conditions of Example 1 are listed in Table 5 below.

**Table 5**

| | Single strand formation of double-stranded DNA | | DNA oxidation step 1-1 |
|---|---|---|---|
| | A volume of reaction solution including 100 ng of double-stranded DNA obtained by Example 1 used for oxidation reaction | Used amount of 1 M NaOH | |
| Example 1 | 26 µL | 4 µL | Performed |
| Comparative Example 9 | 25 µL | 20 µL | Not performed |

As a result, regardless of the presence or absence of hmC in DNA, amplification by PCR did not proceed, and a band of the objective amplification product (177 bp) was not been identified by electrophoresis.

That is, it is considered that when single-stranded DNA was contacted only with oxone without performing the step of contacting single-stranded DNA with polyvalent metal oxide pertaining to the present invention, the single-stranded DNA was degraded.

From the results stated above, it turned out that the single-stranded DNA is required to contact with polyvalent metal oxides pertaining to the present invention, before the single-stranded DNA is contacted with the peroxide pertaining to the present invention or in contact time.

In addition, comparing the experimental results of Comparative Example 9 with Example 1, it was estimated that when sodium tungstate was coexisted before the single-stranded DNA is contacted with oxone or in contact time, the sodium tungstate interacted with DNA to be able to prevent the single-stranded DNA from direct oxidation by oxone, thereby the method of the present invention has become possible. It should be noted that, the results were shown in Table 9 below together with other Examples and Comparative Examples.

### Comparative Example 10: Examination of bringing single-stranded DNA into contact only with hydrogen peroxide

Detection of hmC was carried out but by the same manner as in Example 1 except that DNA oxidation step 1-1 was not carried out, and the condition described below was carried out.

That is, DNA oxidation step 1-2 was carried out by using 30% hydrogen peroxide instead of 50 µL of 300 m M oxone so as to provide 5%, 1% or 0.1% of a final volume concentration in the reaction solution containing single-stranded DNA, and incubated on ice instead of incubating at 60°C.

The differences from the experimental conditions of Example 1 are listed in Table 6 below.

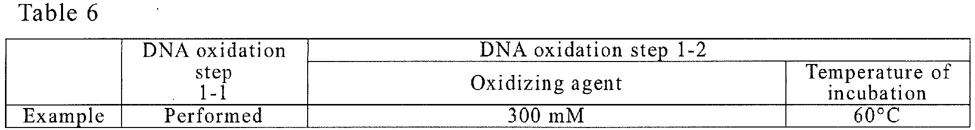

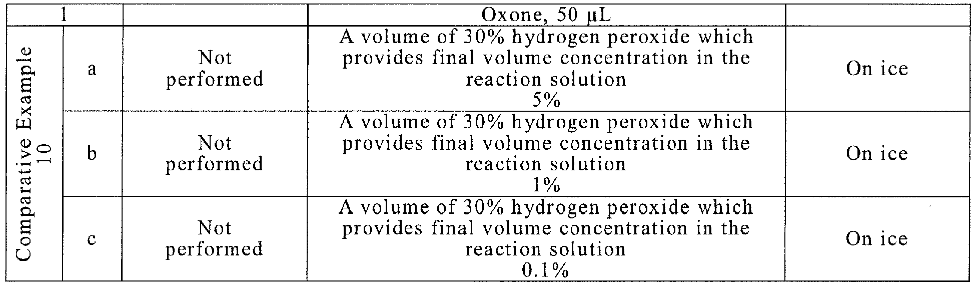

As a result, in all examinations, regardless of the presence or absence of hmC in DNA, amplification by PCR did not proceed, and a band of the objective amplification product (177 bp) was not identified by electrophoresis.

That is, it turned out that without carrying out DNA oxidation step 1-1, in the DNA oxidation step 1-2, when hydrogen peroxide was used as a oxidizing agent, regardless of the concentration of hydrogen peroxide, hmC could not be detected. In other words, it turned out that when a peroxide other than the peroxide pertaining to the present invention is used, hmC cannot be detected.

It should be noted that, the results were shown in Table 9 below together with other Examples and Comparative Examples.

### Comparative Example 11: Examination of bringing single-stranded DNA into contact only with polyvalent metal oxides

Detection of hmC was carried out by the same manner as in Example 1 except for carrying out under the condition described below.

That is, in the single strand formation of double-stranded DNA, the experiments were carried out by using 25 µL of the reaction solution 1 including 100 ng of double-stranded DNA obtained in Experimental Example 1 instead of 26 µL of the reaction solution 1 including 100 ng of double-stranded DNA obtained in Experimental Example 1, and using 20 µL of 1 M NaOH instead of 4 µL of 1 M NaOH, and in the DNA oxidizing step 1-2, by using 50 µL of distilled water (produced by Otsuka Pharmaceutical Co., Ltd.) instead of 50 µL of 300 mM oxone.

The differences from the experimental conditions of Example 1 are listed in Table 7 below.

**Table 7**

| | Single strand formation of double-stranded DNA | | DNA oxidation step 1-2 |
|---|---|---|---|
| | The volume of reaction solution including 100 ng of double-stranded DNA which was obtained by Example 1 used for oxidation reaction | Used amount of 1 M NaOH | Oxidizing agent or Distilled water |
| Example 1 | 26 µL | 4 µL | 300 mM Oxone 50 µL |
| Comparative Example 11 | 25 µL | 20 µL | Distilled water 50 µL |

As a result, regardless of the presence or absence of hmC in DNA, amplification by PCR did not proceed, and a band of the objective amplification product (177 bp) was not identified by electrophoresis.

That is, it turned out that, in the method of the present invention, contact of single-stranded DNA with the peroxide pertaining to the present invention is required, in other words, unless the DNA oxidation step 1-2 is carried out, hmC cannot be detected.

It should be noted that, the results were shown in Table 9 below together with other Examples and Comparative Examples.

### Comparative Example 12: Examination of the use of hydrogen peroxide instead of oxone in Example 6

Detection of hmC was carried out by the same manner as in Example 6 except for carrying out under the condition described below.

That is, in the DNA oxidation step, experiment was carried out by using 30% hydrogen peroxide instead of 50 µL of 300 m M oxone so as to provide 5%, 1% or 0.1% of a final volume concentration in the reaction solution, and by setting the condition as:
(a) single-stranded DNA is contacted with polyvalent metal oxides and hydrogen peroxide at 60°C for 4 hours; or
(b) single-stranded DNA is contacted with polyvalent metal oxides and hydrogen peroxide on ice for 4 hours.

The differences from the experimental conditions of Example 6 were described in Table 8 below.

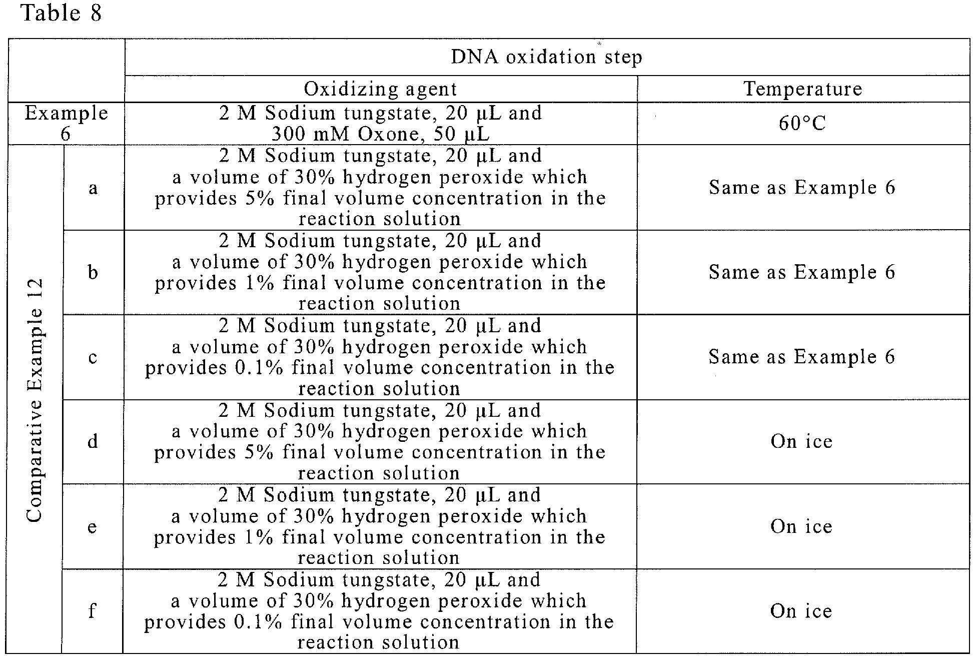

As a result, regardless of the presence or absence of hmC in DNA, a band of the objective amplification product (177 bp) was not identified by electrophoresis.

This was considered to be due to the fact that DNA was degraded by a strong oxidation power of hydrogen peroxide.

That is, it turns out that, even when the polyvalent metal oxide pertaining to the present invention and hydrogen peroxide are contacted simultaneously with single-stranded DNA, hmC in DNA cannot be detected. From the above, it turns out that, hmC in DNA cannot be detected without using both peroxide pertaining to the present invention and peroxide pertaing to the present invention. It should be noted that, the results were shown in Table 9 below together with other Examples and Comparative Examples.

The results of the Examples and Comparative Examples were summarized in Table 9.

It turned out that hmC cannot be detected without using 2 substances of the polyvalent metal oxides pertaining to the present invention and the peroxide pertaining to the present invention, and, that the method of the present invention can be performed by bringing the polyvalent metal oxides pertaining to the present invention and the peroxide pertaining to the present invention into contact with single-stranded DNA either in 2 stage or at the same time. In addition, the method of the present invention could also be used not only for synthetic DNA but also for naturally occurring DNA (genomic DNA).

On the other hand, it turned out that the metal oxides other than the polyvalent metal oxides pertaining to the present invention and the peroxides other than the peroxides pertaining to the present invention cannot detect hmC, in addition, hmC cannot be detected by only either one of the polyvalent metal oxides pertaining to the present invention and the peroxide pertaining to the present invention.

**Table 9**

| | Oxidizing agent in DNA oxidation step 1-1 | Oxidizing agent in DNA oxidation step 1-2 | DNA oxidation step | DNA | Detection of hmC |
|---|---|---|---|---|---|
| Example 1 | Sodium tungstate | Oxone | 2-stage | Synthetic DNA | ○ |
| Example 2 | Potassium tungstate | Oxone | 2-stage | Synthetic DNA | ○ |
| Example 3 | Tungstic acid | Oxone | 2-stage | Synthetic DNA | ○ |
| Example 4 | Sodium molybdate | Oxone | 2-stage | Synthetic DNA | ○ |
| Example 5 | Sodium tungstate | Oxone | 2-stage | Genomic DNA | ○ |
| Comparative Example 1 | Potassium permanganate | Oxone | 2-stage | Synthetic DNA | **×** |
| Comparative Example 2 | Sodium perchlorate | Oxone | 2-stage | Synthetic DNA | **×** |
| Comparative Example 3 | Sodium periodate | Oxone | 2-stage | Synthetic DNA | **×** |
| Comparative Example 4 | 4-methylmorpholine N-oxide | Oxone | 2-stage | Synthetic DNA | **×** |
| Comparative Example 5 | 2-iodobenzene sulfonic acid | Oxone | 2-stage | Synthetic DNA | **×** |
| Comparative Example 6 | Sodium orthovanadate | Oxone | 2-stage | Synthetic DNA | **×** |
| Comparative Example 7 | Copper chloride | Oxone | 2-stage | Synthetic DNA | **×** |
| Comparative Example 8 | Sodium tungstate | Hydrogen peroxide | 2-stage | Synthetic DNA | **×** |
| Comparative Example 9 | Non | Oxone | 1-stage | Synthetic DNA | **×** |
| Comparative Example 10 | Non | Hydrogen peroxide | 1-stage | Synthetic DNA | **×** |
| Comparative Example 11 | Sodium tungstate | Non | 1-stage | Synthetic DNA | **×** |
| | | | | | |

| | Oxidizing agent in DNA oxidation step | | DNA oxidation step | DNA | Detection of hmC |
|---|---|---|---|---|---|
| Example 6 | Sodium tungstate | Oxone | Simultaneous | Synthetic DNA | ○ |
| Comparative Example 12 | Sodium tungstate | Hydrogen peroxide | Simultaneous | Synthetic DNA | **×** |

### INDUSTRIAL APPLICABILITY

According to the method of the present invention, hmC in DNA can be detected easily and accurately.

## Claims

1. A method for detecting a hydroxymethylated cytosine in DNA, which comprises the following steps (1) to (4):
(1): a step in which a single-stranded DNA is contacted with (i) a polyvalent metal oxide or a polyvalent metal acid salt of a metal atom selected from group 6, group 8, group 9 and group 10 of the periodic table, and(ii) a peroxide selected from persulfuric acid, percarboxylic acid and salts thereof;
(2): a step in which a specific region of the single-stranded DNA treated in (1) is subjected to amplification treatment;
(3): a step in which the presence or absence of an objective amplification product obtained in (2) is detected; and
(4): a step in which, on the basis of the results of (3), the presence or absence of the hydroxymethylated cytosine in the specific region of DNA is determined.

2. The detection method according to claim 1, wherein the aforementioned step (1) is a step in which (i) a single-stranded DNA is contacted with a polyvalent metal oxide or polyvalent metal acid salt of the aforementioned metal atom, and then contacted with the peroxide, or a step in which (ii) a single-stranded DNA is contacted with a polyvalent metal oxide or polyvalent metal acid salt of the metal atom and the peroxide simultaneously.

3. The detection method according to claim 1, wherein the metal atom is a metal atom selected molybdenum, tungsten, ruthenium, osmium, rhodium, iridium, palladium and platinum.

4. The detection method according to claim 1, wherein the peroxide is persulfuric acid or a salt thereof.

5. The detection method according to claim 1, wherein the metal atom is metal atom selected from molybdenum, tungsten, ruthenium, osmium, rhodium, iridium, palladium and platinum, and the peroxide is persulfuric acid or a salt thereof.

6. The detection method according to claim 1, wherein the metal atom is molybdenum or tungsten.

7. The detection method according to claim 1, wherein the metal atom is molybdenum or tungsten, and the peroxide is persulfuric acid or a salt thereof.

8. A reagent kit for detecting the hydroxymethylated cytosine in DNA comprising(i) a reagent including a polyvalent metal oxide or a polyvalent metal acid salt of a metal atom selected from group 6, group 8, group 9 and group 10 of the periodic table, and(ii) a reagent including a peroxide selected from persulfuric acid, percarboxylic acid and the salts thereof.

9. The reagent kit according to claim 8, wherein the metal atom is a metal atom selected molybdenum, tungsten, ruthenium, osmium, rhodium, iridium, palladium and platinum.

10. The reagent kit according to claim 8, wherein the peroxide is persulfuric acid or a salt thereof.

11. The reagent kit according to claim 8, wherein the metal atom is the metal atom selected molybdenum, tungsten, ruthenium, osmium, rhodium, iridium, palladium and platinum, and the peroxide is persulfuric acid or a salt thereof.

12. The reagent kit according to claim 8, wherein the metal atom is molybdenum or tungsten.

13. The reagent kit according to claim 8, wherein the metal atom is molybdenum or tungsten, and the peroxide is persulfuric acid or a salt thereof.


SEQUENCE LISTING

<110>  WAKO PURE CHEMICAL INDUSTRIES, LTD.

<120>  Detection method for hydroxymethylated cytosine and detection kit
        thereof

<130>  1899

<160>  9

<170>  PatentIn version 3.3

<210>  1
<211>  566
<212>  DNA
<213>  lambda phage

<400>  1
gcaacatgaa taacagtggg ttatccaaaa ggaagcagaa agctaaatat ggaaaactac      60

aatacgatgc cccgttaagt tcaatactac taatttttag atggaaaacg tatgtaatag     120

agagtaactt aaaagagaga tcctgtgttg ccgccaaata aattgcggtt attttaataa     180

aattaagggt tactatatgt tggagtttag tgttattgaa agaggcgggt atattcctgc     240

agtagaaaaa aataaggcat tcctacgagc agatggttgg aatgactatt cctttgttac     300

aatgttttat cttactgtct ttgatgagca tggtgaaaaa tgcgatatcg gaaatgttaa     360

aattggtttt gtaggtcaaa aagaagaagt aagcacttat tcattaatag ataaaaaatt     420

cagtcaactc cctgaaatgt tttttccctt aggtgaaagc attgactact atgttaatct     480

cagcaaatta agcgatggtt ttaaacataa ccttcttaaa gctattcagg atttagtagt     540

atggccaaat cgattagccg acattg                                          566


<210>  2
<211>  26
<212>  DNA
<213>  Artificial

<220>
<223>  Primer

<400>  2
gcaacatgaa taacagtggg ttatcc                                           26


<210>  3
<211>  23
<212>  DNA
<213>  Artificial

<220>
<223>  Primer

<400>  3
caatgtcggc taatcgattt ggc                                              23

<210>  4
<211>  177
<212>  DNA
<213>  Artificial

<220>
<223>  Oligonucleotide

<400>  4
gttggagttt agtgttattg aaagaggcgg gtatattcct gcagtagaaa aaaataaggc     60

attcctacga gcagatggtt ggaatgacta ttcctttgtt acaatgtttt atcttactgt    120

ctttgatgag catggtgaaa aatgcgatat cggaaatgtt aaaattggtt ttgtagg       177


<210>  5
<211>  27
<212>  DNA
<213>  Artificial

<220>
<223>  Primer

<400>  5
gttggagttt agtgttattg aaagagg                                         27


<210>  6
<211>  25
<212>  DNA
<213>  Artificial

<220>
<223>  Primer

<400>  6
cctacaaaac caattttaac atttc                                           25


<210>  7
<211>  163
<212>  DNA
<213>  Artificial

<220>
<223>  Oligonucleotide

<400>  7
gctccagtgt agacatacaa tagaccactc gtccctgtgg ctccgggcag cagcctcatc     60

tgagaccctc ctgagacatc tcgtgcaggg cagccgtagt gtgtggcttc cccagggctg    120

ctctaacaga tcaccatcct tgccatggct taagaagctg cag                      163


<210>  8
<211>  26
<212>  DNA
<213>  Artificial

<220>

<223>  Primer

<400>  8
gctccagtgt agacatacaa tagacc                                          26


<210>  9
<211>  21
<212>  DNA
<213>  Artificial

<220>
<223>  Primer

<400>  9
ctgcagcttc ttaagccatg g                                               21